# EUROPEAN PATENT SPECIFICATION

(11) **EP 1 009 338 B1**
(45) Date of publication and mention of the grant of the patent: **04.10.2006**
(21) Application number: 98941086.5
(22) Date of filing: 27.08.1998
(51) Int. Cl.: A61F 2/44

(54) **CORTICAL BONE CERVICAL SMITH-ROBINSON FUSION IMPLANT**
KORTIKALKNOCHENIMPLANTAT FÜR WIRBELFUSION NACH SMITH-ROBINSON
IMPLANT DE SUBSTANCE CORTICALE POUR DES PROCEDES DE FUSION DES VERTEBRES CERVICALES DE SMITH-ROBINSON

(30) Priority: 27.08.1997 US 920630
(43) Date of publication of application: 21.06.2000
(60) Divisional application: 06017260.8
(73) Proprietor: Regeneration Technologies, Inc., Alachua, FL 32616-2650 (US); Medtronic Sofamor Danek, Inc., Memphis, TN 38132 (US)
(72) Inventor: GROOMS, Jamie, M., Alachua, FL 32615 (US); CARTER, Kevin, C., Alachua, FL 32615 (US); SANDER, Tom, Alachua, FL 32615 (US); DULEBOHN, David, H., Naples, FL 33940 (US)
(74) Representative: Samuels, Lucy Alice
(86) International application number: PCT/US1998/017769
(87) International publication number: WO 1999/009914

(56) References cited:
- WO-A-97/14378
- WO-A-97/25945
- WO-A-98/17209
- US-A- 3 848 601
- US-A- 4 950 296
- US-A- 5 728 159

## Description

This invention relates to a cortical bone implant for use in cervical Smith-Robinson vertebral fusion procedures, as well as methods for the manufacture and use thereof.

### 1.2 Background Art:

Since at least the mid to late 1950's anterior cervical spinal fusions have been performed in order to alleviate chronic neck, arm and shoulder pain caused by trauma, disc herniation, or spondylosis (Robinson and Smith, 1955; Smith and Robinson, 1958). The classic procedure referred to as the Smith-Robinson cervical fusion employs a horseshoe-shaped graft to promote vertebral fusion (Robinson *et al.,* 1962). The Cloward technique employs a cancellous bone dowel (Cloward, 1958), and the Bailey-Badgley procedure uses a strut (Bailey and Badgley, 1960). In a study comparing the compressive load capacity of the various implants used according to these procedures, it was found that the Smith-Robinson graft could sustain loads up to 344 N, the Cloward dowel could sustain loads of up to 188 N, and the Bailey-Badgley type could sustain loads up to 195 N, (White and Hirsch, 1972). In a modified Smith-Robinson procedure, the horseshoe-shaped implant is inserted with the cortical end of the implant located posteriorly, which has been reported to increase the fusion rate while decreasing the graft extrusion and collapse sometimes experienced with the Cloward dowels (Whitecloud and Dunsker, 1993). However, in a recent study evaluating the success and relief rates achieved according to these procedures, it was found that less than 100% success rate (fusion, patient improvement and absence of complications) was achieved, regardless of which method or implant was used (Grooms *et al,* 1996).

U.S. Patent No. 5,306,309, discloses a spinal disk implant comprising a solid body of biocompatible synthetic material arranged to define a right-rectangular solid having two opposed side faces and two opposed transverse faces, including a convexly curved anterior face and a posterior face, for implantation in the intervertebral space. The discussion of vertebral and intervertebral morphology is hereby incorporated by reference.

U.S. Patent No. 5,609,635, discloses a lordotic interbody spinal fusion implant comprising a wedge shaped metallic cage for insertion into the intervertebral space.

U.S. Patent No. 5,306,307, discloses a ceramic spinal disk implant having a serrated edge.

None of these references disclose a cortical bone intervertebral implant having a substantially "D"- or bread-loaf-shaped structure having a canal into which osteogenic, osteoinductive, or osteoconductive materials may be packed, which sustains spinal loads, and which is remodeled into the spine in the course of fusion. Accordingly, the present invention addresses the need in the art for improvements to both the implant and the avoidance of post-surgical complications from anterior cervical fusions. The present invention provides a new cortical bone implant for use in achieving anterior cervical fusions when implanted according to the Smith-Robinson procedure. In addition, in view of the peculiar characteristics of bone, the present invention comprises unique methods and apparatuses for the manufacture of the substantially "D"-shaped cortical bone implant.

US 5, 728, 159 describes an implant having cross-sections of "elongate cortical bone". The hole in the centre of these implants is the natural intra medullary canal of the bone having natural walls, wherein the canal would ordinarily contain the bone marrow in the living tissue.

### 2.0 Summary of the Invention

An implant as defined in claim 1 composed substantially of cortical bone is provided for use in cervical Smith-Robinson vertebral fusion procedures. According to methods useful in this invention, the implant is derived from allograft or autograft cortical bone sources, is machined to form a substantially "D"- or other appropriately shaped implant having a canal into which osteogenic, osteoinductive, or osteoconductive material may be packed. The method of the invention in defined in claim 14. The implant can be inserted into the space between adjacent cervical vertebrae to provide support and induce fusion of the adjacent vertebrae.

### 3.0 Brief Description of the Drawings

Figure 1 provides several views of the fusion implant of this invention.
Figure 2 provides views of the core cutter and drill assembly and the bone plug formed by cutting into the diaphysis of a long bone when such a core cutter and drill assembly is used.
Figure 3 provides a view of broach as used according to this invention and an asymmetric canal formed by use of such a broach.
Figure 4 provides several views of an apparatus for machining a profile on the exterior surface of the implant of this invention.
Figure 5 provides a view of an apparatus for inscribing retention teeth in the upper surface, lower surface or both upper and lower surfaces of the implant.
Figure 6 provides several views and dimensions for specific embodiments of the implant of this invention.
Figure 7 provides a view of a stacked embodiment of the implant of this invention.
Figure 8 provides several views of an implant of this invention formed by juxtaposition of mirror image halves of the implant, as well as an embodiment useful for posterior lumbar intervertebral fusion procedures (PLIFs).
Figure 9 provides a view of a stacked embodiment of the implant of this invention wherein the stacked constituents thereof are retained in registered relationship by press-fitting or otherwise bringing more than one implant into contact with each other and having a cancellous plug or other biocompatible material located in the central canal of each stacked implant, thereby acting as a retention pin.
Figure 10 shows an alternate method for producing bone stock for making the implant of this invention of essentially unlimited height from the anterior margin of the tibia or the linea aspera of the femur.
Figure 11 shows dimensions and further processing of the implant of this invention produced according to the alternate method of figure 10.
Figures 12-17 show final profiles for implants produced according to the alternate method of figures 10 and 11.

### 4.0 Detailed Description of the Invention

According to this invention, a substantially "D"-shaped cortical bone implant for cervical Smith-Robinson fusions is produced, preferably under aseptic conditions. Class 10 clean room processing is desirable, and sterilization of all machining tools is likewise preferred, (particularly after switching from one allograft donor to the next), so that the finished product may be treated by standard techniques known in the art (alcohol, peroxide, or like treatments), prior to storage and shipment to physicians for use in implantation procedures. Because of the peculiarities of working with bone, and in particular, because of the desirability of maintaining aseptic conditions while working with this material, novel approaches have been adopted in the production of the product of this invention.

The implant is preferably formed from cortical bone obtained from tibia, femur or other source of strong cortical bone. The bone source may be autograft or, due to possible complications at the donor site (infection, pain, delayed healing), is preferably, allograft bone. In addition, it is critical that the source bone be derived from a donor whose medical history is well known (absence of transmissible diseases, cancer, osteoporosis), and that the donor bone be obtained under aseptic conditions according to accepted practices in the art of tissue banking. In addition, extensive *in vitro* testing should be conducted to ensure the absence of pathogenic agents.

The approach adopted in describing the implant of this invention is to first provide a narrative disclosure of preferred methods for making the implant, followed by a detailed description of the implant itself, followed by a detailed description of various apparatuses and aspects of the machining process, and finally, a detailed description of the method of using the implant.

### 4.1 Narrative Description of Implant Manufacture

While any shape of cortical bone may be used to begin with, we have found that for consistent production of cortical bone which may be reliably machined, it is advantageous to commence with a plug of bone which extends from the exterior of the diaphysis of a long bone toward the intramedullary canal (where, *in vivo,* the bone marrow resides). The result is a bone plug or dowel which has an outer substantially cortical end and an internal end which is composed largely of soft cancellous bone. In cutting the bone plug, we have discovered that the use of a core cutter is convenient. This device comprises an outer coring element of any desired diameter, whereby the diameter of the bone plug is defined, and a centrally located solid drill bit, which provides a canal through the center of the bone plug as well as stability for the core cutting element. The core cutter-drill assembly is preferably torqued by an air drill, driven by sterile air, and the source bone is preferably immobilized in a sterilized vice during the core-cutting process.

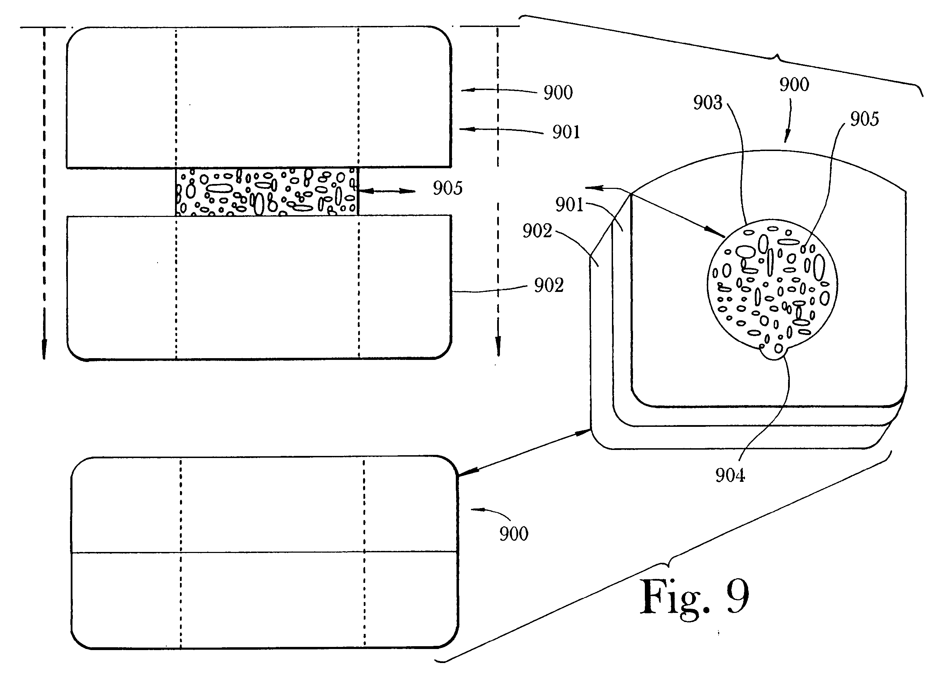

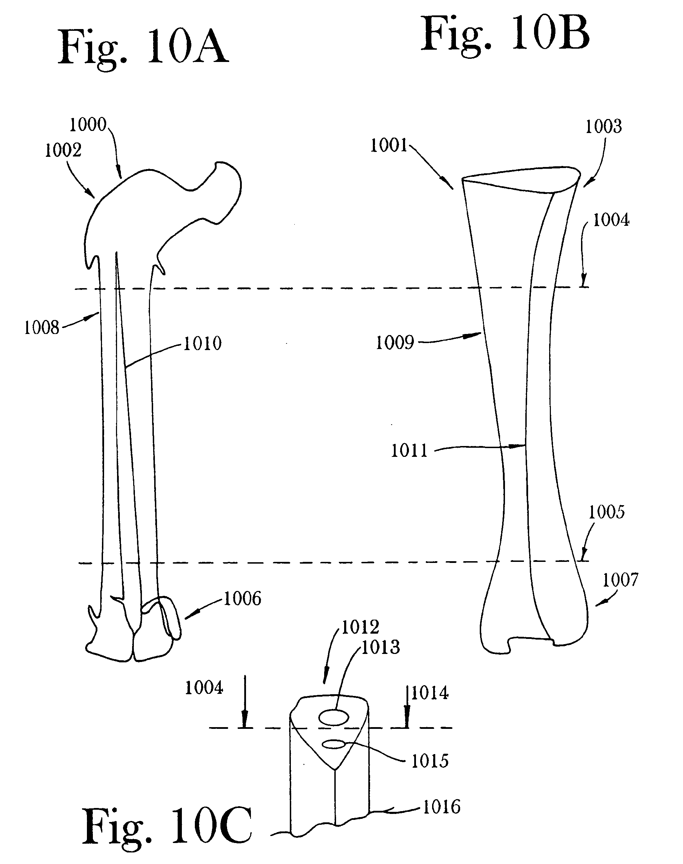

We have discovered that in the above-described manner, cortical bone implants may be fashioned having heights, widths and lengths which are practically useful in the Smith-Robinson cervical fusion method. According to this method, the height of the implant is only limited by the distance from the exterior of the bone diaphysis to the intramedullary canal. However, we have discovered that, by this method, final implant heights from about 7 mm to about 14 mm may be produced, depending on the choice of bone source and the location on the bone from which the bone plug is cored. Since it is extremely rare for the cervical intervertebral space to extend beyond these limits, this method is therefore capable of supplying implants of required or useful heights. Likewise, the length and width of the implant are defined by the diameter of the core-cutter, and final lengths and widths of between about 7 and 14 mm are easily provided for by this method. In addition, where the need arises for heights between about 10 mm and 14 mm, or if difficulty is experienced in obtaining donor bone having a sufficient width from the exterior of the bone to the intra-medullary canal to provide such heights, alternate methods of producing the implant of desired heights disclosed herein may be employed. For example, in a first such alternate method, implants of this invention are produced and then stacked to provide a unitary implant of the desired height dimensions. Such stacked implants may be maintained in a unitary association by drilling appropriate holes through the height of the implant, and inserting therein appropriate retention pins made from any desirable material, including cortical bone, bioabsorbable synthetic polymer, titanium or other metallic retention pins. Alternatively, the stacked implants may be retained in a unitary association by means of a plug of cancellous bone, hydroxyapatite or other biocompatible, osteoconductive or osteoinductive material, and press-fitting the stacked implants to achieve the desired height (see figure 9). In a further alternate method, a section of cortical bone along the long axis of a long bone may be machined according to methods known in the art. By then further shaping and cutting appropriate heights in such cortical bone, and bringing halves of the implant into juxtaposition with each other, implants of any desired shape and height are produced. In yet a further alternate procedure, (see figures 10-17), unitary implants of this invention of essentially unlimited height are produced by lengthwise sectioning the anterior margin of the tibia or linea aspera of the femur, segmenting the substantially triangular cortical bone to desired heights, drilling a cannulation through the segments thus produced, and finally shaping the implants to desired dimensions, as defined below for the first principal method of making the implant of this invention.

Continuing with a description of the first method for making the implant of this invention, the cancellous bone on the internal side of the bone plug is removed by any convenient means, including with a saw, an abrasive means such as a diamond tipped rotary sander, or a tooling bit mounted in a lathe, to produce a "washer" shaped piece of substantially cortical bone. Both the internal and external ends of the bone plug should be machined flat, thereby forming a top face and a bottom face, each of which is substantially planar, and preferably parallel. While the cancellous bone is partially or completely removed by this process, there remains a slight difference in the density of the bone from the external (cortical) to the internal (cancellous or originally intra-medullary) aspect of the bone plug. It is desirable to record the orientation of the bone plug as subsequent machining steps proceed most efficiently when machined from the external aspect toward the internal aspect.

In order to accommodate subsequent machining steps and to provide an orientation to the implant according to which the surgeon may properly insert the implant, the circular internal canal formed by the centrally located solid drill bit of the core-cutter is modified to form an asymmetric shape, such as a key way. This may be achieved by any of a number of different means, including drilling a slot into an aspect of the internal canal closest to the external (more dense cortical) end of the dowel. In one embodiment of this invention, we have found that an implant of consistently good final quality may be machined by conversion of the circular canal into a substantially "D" shaped canal having three essentially rectangular walls and a fourth convexly curved wall. We have found that it is desirable for the curvature of the convexly curved wall to approximate the external curvature of the bone plug. This modification may be achieved by any of a variety of means. However, we have invented an efficient means by which consistently usable implants may be reproducibly machined. This is accomplished by immobilizing the implant, for example in an arbor press assembly, and, preferably from the originally cortical external (denser) end of the implant, slowly forcing a broach through the originally circular canal. The broach is preferably a hard metallic member having a plurality of spaced-apart ribs or rings machined therein, with indentations provided between each ring which thereby form the spacing between adjacent rings. In addition, the edges of each ring are desirably very precise, angular, and sharp, such that as the broach is forced through the originally circular internal canal, the sharp cutting edge of each ring shaves off an incremental amount of bone as the ring passes through the implant. Each ring of the plurality of rings has a shape which, starting at the insertion end of the broach is tapered from an essentially circular shape to any desired final shape for the canal. Accordingly, in one embodiment of this invention, the rings transition from a circular shape to a substantially "D"-shaped profile over several inches and over a plurality of spaced apart rings. It will be appreciated that the length of the broach and the number of rings used is defined by the amount of bone that must be removed to form the new shape, the width of each ring and the width of the space between each ring. Removal of no more than about 0.004" of bone by each ring has been found to be a sufficiently small transition to ensure that the vast majority of implant blanks survive this machining step. Broaches of approximately 6" in length have been found adequate for most implant shapes, but for very asymmetric shapes (e.g. an implant which is 11 mm wide and 14 mm long), more bone would need to be removed to form the "D"-shaped canal than from a symmetric implant (e.g. a 14 mm wide by 14 mm long implant). This need may be accommodated by use of more than one broach, with the shape of the insertion end of each consecutive broach substantially matching the shape of the last ring on the previous broach.

Having formed an asymmetric shape, such as a key way, from the internal canal running through the implant, we have found it desirable to modify the external profile of the implant from a substantially circular shape to another desired form. In this invention, the external form of the implant is machined so as to proportionately match the shape of the substantially "D"-shaped internal canal. An external "D"-shaped profile has been used in implants known in the art (see for example U.S. patent 5,306,309; 5,522,899) made from materials other than bone, because of the ability of the convexly curved face of the implant to substantially match the curvature of the anterior aspect of the intervertebral disk into which the implant is to be inserted, as well as to provide efficient spinal load distribution over the remainder of the implant. However, due to the peculiar nature of bone, and the requirements of aseptic or sterile manufacturing, inventive methods and apparatuses were required to produce the desired external profile for the cortical bone implant. It will be recognized that, based on the instant disclosure, a substantially "D"-shaped external profile of the implant may be machined by a variety of means which vary from the precise methods disclosed herein. In addition, other external profiles than the "D"-shaped profile are likewise enabled by modifications of the methods and apparatuses disclosed herein for formation of the "D"-shaped external or internal profile. Thus, according to one alternate method of making the implant of this invention, for example where the linea aspera of the femur is sectioned or where the anterior margin of the tibia is sectioned, an external profile that substantially varies from a "D"-shaped device may be produced, (see figures 10-17).

We have found it convenient and reproducible to use either of two principal methods for machining the external profile. The implant, with the "D" or alternately shaped internal canal being used as a key way, is fitted onto the end of a spindle which precisely matches the shape of the internal canal of the implant, thereby providing purchase for machining of the external profile of the implant. In a first preferred method, as the implant is rotated on the spindle, it is contacted with an asymmetric generator (grinding) wheel attached to a cog which meshes at a known registration point with a cog to which the spindle with the implant is attached. The speed of rotation of the exterior of the spindle mounted implant, and the exterior of the generator wheels are designed to differ such that as the generator wheel and implant are contacted and are rotated in fixed registration, the generator surface (which is preferably an abrasive diamond plated surface), grinds bone from the external surface of the implant, to form a profile thereon defined by the asymmetric shape of the grinder wheel.

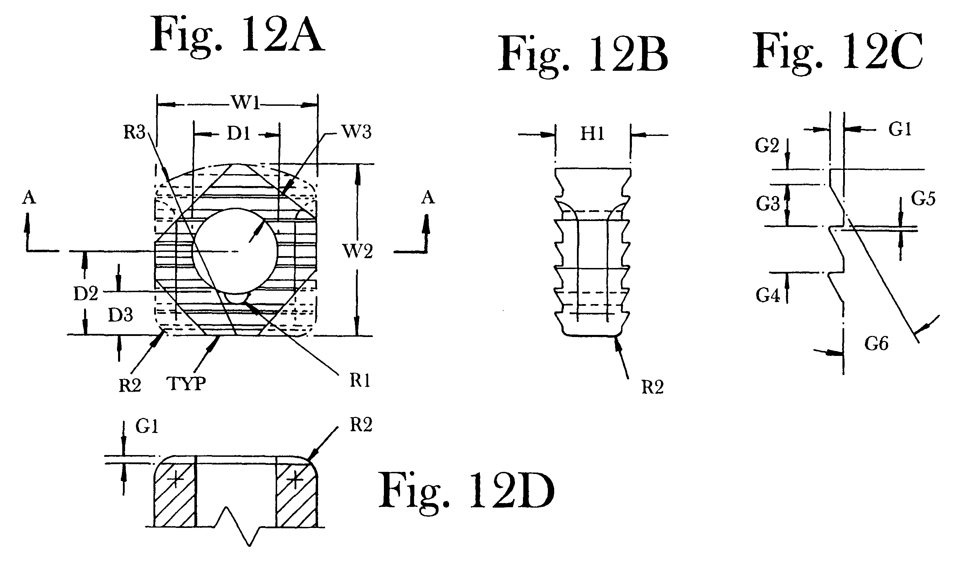

In a second external profile generation method, the implant, with the "D" or alternately shaped internal canal being used as a key way, is fitted onto the end of a spindle which precisely matches the shape of the internal canal of the implant, thereby providing purchase for machining of the external profile of the implant. In this method, the spindle is affixed to an asymmetric cam which rotates concentrically with the spindle, and therefore the implant. The thus mounted implant is contacted with a cutter means, such as a sharp bit having cutting edges which rotate about an adjacent axis. The implant mounted spindle riding on the asymmetric cam is biased to contact the rotating cutter, which thus traces a profile onto the exterior of the implant defined by the shape of the asymmetric cam. For purposes of this disclosure, use of the term "asymmetric cam" should be understood to mean any desirable shape such that upon production of the implant, the shape thereof is defined by that of the asymmetric cam. Shapes contemplated by this disclosure include, but are not limited to, elliptical shapes, D-shapes, partially curved shapes, and the like. The implants produced according to any of the alternate procedures are likewise shaped, although the final shape may vary depending on the size of the bone stock used (see for example the final shapes of the device shown in figures 12-17).

Once the external profile has been machined, the implant is removed from the spindle, an external feature may be machined into the upper and lower surfaces to prevent backing out of the implant upon insertion into the intervertebral space. This may be achieved by a number of means, such as by machining teeth into the upper, lower, or both surfaces of the implant. In a preferred embodiment of this invention, the implant is passed through a set of opposing jaws bearing teeth which broach a tooth-shaped profile into the implant as it is forced through the jaws. Alternatively, the implant is passed several times over a ridged surface which cuts the desired tooth profile into the upper, lower or both surfaces of the implant. Preferably, the thus formed teeth angle toward the anterior (convexly curved) face of the implant to prevent backing out of the implant once it is inserted into an appropriately shaped cavity formed in the intervertebral space in an anterior aspect of the cervical spine. In order to accommodate the difficulty surgeons experience in forming precise angles when forming such cavities in the spine, (see for example U.S. patent No. 5,397,364 disclosing a beveled edge to reduce trauma upon insertion of a metallic spinal implant), a beveled edge of defined radius is preferably machined into three faces of the implant, but leaving the anterior face unbeveled. The sharp anterior edge, like the teeth in the upper and lower surfaces of the implant, retards backing out of the implant.

### 4.2 Detailed Description of the Implant

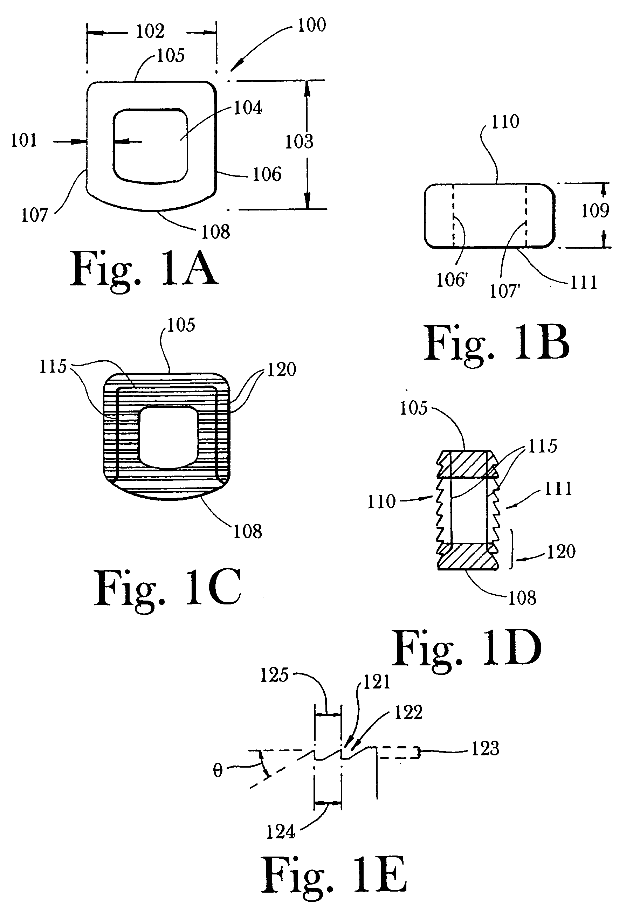

Referring now to figure 1A, there is shown a top view, as if viewed from the top of the spinal column, of a substantially "D"-shaped cortical bone implant **100.** The implant has a wall thickness **101,** a length **103,** a width **102,** and an internal canal **104,** which fall within desired tolerances (see discussion below). The implant comprises four contiguous walls, including a substantially straight rear wall 105, substantially straight side walls **106** and **107,** and a preferably curved front wall **108.** In figure 1B, there is shown a side view of the implant **100,** revealing the height **109,** of the implant. In addition, this view shows, in outline, the internal side walls **106'** and **107'** of the internal canal, **104.** It also shows the top **110** and bottom **111** surfaces of the implant. In figure 1C, there is shown a top view of an embodiment of the implant **100** in which an external feature **120** has been inscribed onto the top **110** and bottom **111** surfaces of the implant. In addition, a "radius" or bevel **115** is shown on the two side and posterior edges of the implant. Figure 1D shows a side view of the implant **100** in which the inscribed feature **120** can clearly be seen in the top **110** and bottom **111** surfaces of the implant. In this view, it can be seen that the external feature **120** has the side profile of a set of teeth, all of which angle toward the anterior face **108** of the implant. An outline of the bevel **115** is also evident in this view, as is the rounded posterior edge 105. As can be seen, in this embodiment of the invention, the anterior edge **108** is maintained with a sharp edged. In figure 1E, there is shown a detail of one embodiment of the inscribed feature **120** on the portion of the implant indicated in figure 1D. In a preferred embodiment, the feature **120** defines a tooth-like structure, with teeth **121** separated from each other by concavities **122.** An angle θ defines the grade of the concavity as it ramps to the tooth. The tooth height **123,** space between teeth **124,** and aperture of the concavity **125** may all be defined by the manufacturer to optimize retention of the implant within the cervical spine after proper placement.

### 4.3 Detailed Description of the Method of Manufacturing the Implant

Because of the peculiar nature of bone, and the desirability of sterile or aseptic manufacturing, specific and specialized procedures and apparatuses are required for successful formation of the implants of this invention. Those skilled in the art will recognize that, based on the methods and apparatuses disclosed herein, the implant of this invention may be manufactured by alternate means suggested by those described herein. Nonetheless, through careful design and knowledge of bone structure, instruments for the manufacture of the implant of this invention have been invented for this purpose. In what follows, specific details with respect to preferred method and apparatuses for making the implant of this invention are provided. It should be recognized that the invention should not be construed as being limited to these specifics.

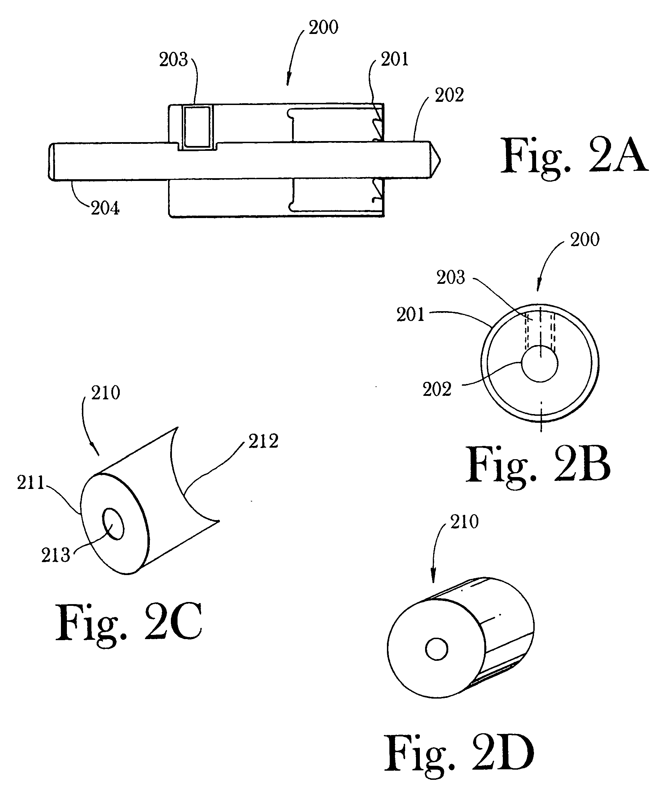

Referring to figure 2, there is shown in side view in figure 2A a core cutter **200,** having a core bit 201 which is affixed by a set screw **203** to the shaft **204** of a drill bit **202,** centrally located within and coaxial with the core cutter. In figure 2B, an end-on view of the core cutter **200** is provided showing the set screw **203** in outline. Figure 2C shows a side view of the bone plug **210** which is formed by cutting a plug of bone from the diaphysis of a long bone using the core cutter **200.** At one end, **211,** originally the external cortical surface of the bone shaft, there is a substantially cortical bone surface through which a hole **213** is formed by the central bit **202** of the core cutter **200.** The other end, **212,** is an irregular and bone surface which, *in vivo,* formed part of the wall of the intramedullary canal. Cancellous bone or other microstructure at the end **212** is removed, and both ends are ground, cut or otherwise machined to be substantially flat and parallel, to form the substantially cortical bone plug **210** shown in figure 2D.

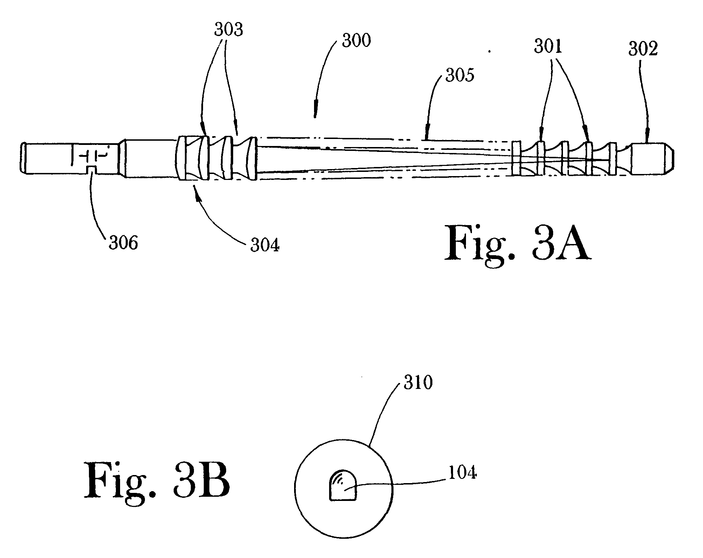

Referring to figure 3, there is shown in figure 3A an internal canal profile broaching tool **300.** A plurality of spaced-apart ribs or rings **301** are provided along the length of the broach which taper from a substantially circular shape at the insertion end **302** of the broach, to substantially "D"-shaped rings **303** (or any other desired shape) at the completion end **304** of the broach 300 (intermediate ribs **305** are not shown; rather, the outline of the taper angle is shown). A notch or groove **306** is provided in the broach completion end **304** for releasably affixing the broach into a means, such as a press, for forcing the broach through the implant canal. In figure 3B, there is provided an end-on view of the cancellous bone plug **310** after the broaching procedure is completed. As can be seen, the internal canal **104** has been converted from a circular canal into a substantially "D"-shaped canal. As will be appreciated from this disclosure, any of a number of different asymmetric shapes in the internal canal **104** may be defined by this or analogous means, the principal goal being to provide a purchase (referred to herein as a "key way") within the implant for external machining of the implant. In one embodiment (see figure 9), the canal may be retained as a substantially circular canal, and a slot **904** is machined therein to provide the necessary asymmetry to form a key way.

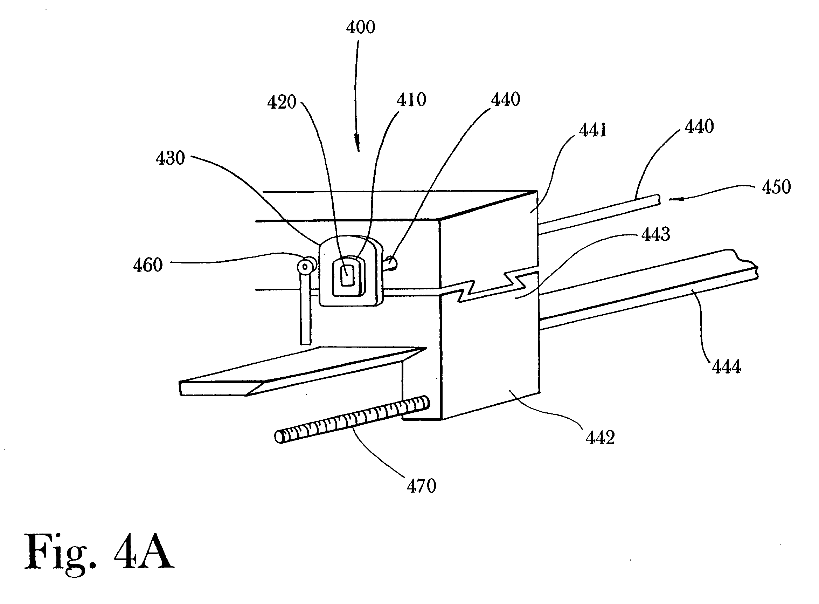

Having formed a key way within the implant, it is possible to modify the external profile of the implant. In one aspect of this invention, referring to figure 4A, this is conveniently achieved by affixing the implant **410** to the spindle **420** of a lathe **400.** The spindle shaft **440** extends, through bearings (not shown), to a means **450** (such as a handle or a motor) for rotating the spindle. Affixed to the spindle-shaft is a cam **430,** the shape of which defines the ultimate external profile of the implant **410.** The spindle shaft **440** and bearings are mounted in a cross slide **441,** which translates in a first plane, referred to as the "Y-plane". Motion in the Y-plane is limited by contact of the cam **430** with a limiting means **460** such as a cam follower, which remains in register with a carriage **442** which translates along a plane, the "X-plane", transverse to the Y-plane motion of the cross-slide. The cross-slide is mounted in a slide-way **443** of the carriage **442,** which in turn is slideably mounted on the bed **444** of the lathe, such that the carriage **442** is permitted to translate along the X-plane. Travel of the slide **442** along the X-plane is limited by means of a stop screw **470.**

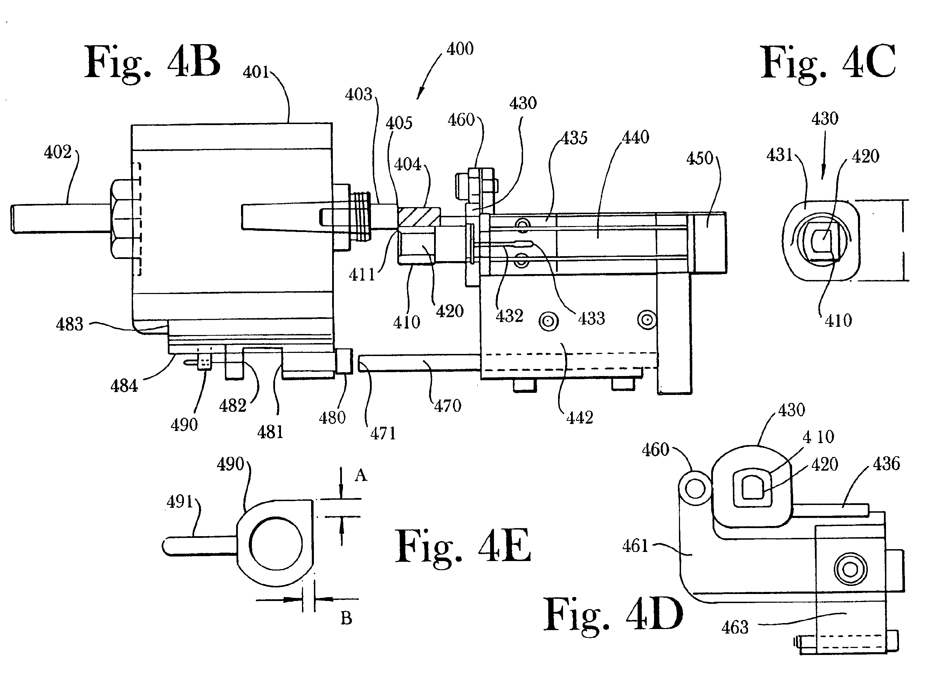

Further detail of this means for generating the external profile of the implant is provided in figure 4B, which provides a side view of one specific embodiment of the implant external profile generator **400.** An air driven turbine within housing **401** provides a source of torque to turn a shaft **402.** A means for cutting or grinding the external surface of the implant **410,** such as an appropriately fashioned cutter or bit having a non-cutting end **403** for fixation to the shaft **402.** Extending from the non-cutting end **403** which has a first diameter, is a cutting surface **404,** having a second, smaller diameter. A "shoulder" **405,** forms a radius extending between the smaller diameter of the cutting surface **404** and the larger diameter of non-cutting surface **403.** The cutting surface **404** is contacted with the implant blank **410,** mounted on spindle **420,** to which, as described above is mounted an asymmetric cam **430.** The thus mounted implant blank **410** is brought into contact with the cutting surface **404,** by virtue of translation in the X-plane of the carriage **442.** The spindle **420,** and thus the asymmetric cam **430** are rotated, manually or by motor driven means, through shaft **440** and handle **450** which are attached concentrically with the cam **430.** Preferably, the asymmetric cam **430** is elastically biased toward a stationary cam follower **460.** In this fashion, after several revolutions of the handle **450,** the shape of the asymmetric cam **430** generates the desired external profile of the implant **410** riding on the spindle **420,** through contact with the rotating cutting surface **404.**

To ensure that the implant blank is machined only up to the point that the forward edge **411** of the implant approaches but does not contact the "shoulder" **405** on the cutter, a stop screw **470** is provided. The stop screw **470** is set to prevent further advancement of the implant blank **410** by stopping advancement of the carriage **442** when the leading edge **471** of the stop screw comes into contact with a measuring screw **480.** The appropriate setting of the stop screw **470** is achieved at the start of the milling process by first placing the implant **410** between the end **481** of the measuring screw **480** and an anvil **482,** and tightening the measuring screw **480** until it just makes contact with the implant. In this fashion, the measuring screw **480** and anvil **482** essentially form a micrometer, with the gap being defined by the width of the implant. Both the measuring screw **480** and anvil **482** are housed within a measuring slide **483** which, when slid all the way to the left as shown in figure 4B, abuts a rotateable stop cam **490,** retained within the same slide-way as the measuring slide **483** by a retainer **484.** The rotateable stop cam 490 may be set in either of two positions, which produces a difference in the stopping point of the stop screw **470** of approximately 0.06". The significance of this difference is that the first position arrests advancement of the stop screw **470** (and therefore the carriage **442**) just before the implant **410** contacts the radius shoulder **405** of the cutting surface **404.** In the second position, the stop cam **490** allows the stop screw to advance the additional approximately 0.06" to allow contact of the implant **410** with the shoulder **405** of the cutting surface **404** to thereby bevel the edges of the implant **410** that are thus contacted. Accordingly, in the pre-milling setup, the stop cam **490** should be rotated such that the stop screw **470** is forced to stop the extra 0.06", following which a further processing step may be carried out in which the stop cam **490** is rotated to the second position in which the stop screw **470** is allowed to advance this additional approximately 0.06".

In figure 4C, there is provided an end-on, rear view (i.e. looking from the handle **450** toward the spindle **420)** of the asymmetric cam **430,** the spindle **420** and the implant **410.** In addition, in this detail view, an additional feature in the asymmetric cam **430** is seen as a diminution in the thickness along three faces **431** of the asymmetric cam **430** which is a relief in the rear of the asymmetric cam **430.** The significance of this relief **431** is that it restricts the contact of the implant **410** with the shoulder **405** to the extent defined by the relief in the rear of the asymmetric cam **430.** As noted above, in fashioning an implant site in the intervertebral space during a partial discectomy, surgeons are unable to produce perfectly sharp angles. To accommodate this imperfection, to prevent trauma upon insertion of an implant with sharp edges, and to create as tight-fitting an implant as possible, the fashioning of a bevel around the edges of the implant that are inserted into the intervertebral space created by the surgeon is desired. At the same time, in order to prevent backing out of the implant, it may be desirable to retain a sharp anterior implant edge, and therefore the relief in the cam **430** does not extend completely around the cam. Thus, upon completion of the external profile of the implant **410** as described above, the carriage **442** is backed away from the cutter, the stop cam **490** is flipped to its second position allowing advancement of the stop screw **470** the additional approximately 0.06" mentioned above. At the same time, a shot pin **432** is advanced into the relief **431** by means of a shot pin mover **433,** thereby allowing rotation of the cam **430** only to the extent permitted by the shot pin **432** as it rides within the relief **431.** With the shot pin **432** riding in the relief **431,** the "shoulder" **405** contacts the leading edge 411 of the implant blank **410,** thereby rounding three edges of the implant **410.** After machining the leading edge **411** of the implant **410,** the implant is removed from the spindle **420**, turned around, and re-positioned on the spindle **420**, to inscribe the bevel on three edges of the other side of the implant.

In figure 4D, a frontal view is provided of the spindle **420,** the implant **410,** the asymmetric cam **430,** and the cam follower **460.** Also shown is the cam adapter **461,** by means of which the cam follower **460** is affixed to the carriage **442,** and by means of which the cam follower **460** maintains the cutting surfaces **404/405** in contact with the implant **410** as defined by the shape of the asymmetric cam **430.** Also shown is a part of the cross-slide **441,** which is preferably biased or which may be pushed manually toward the cam follower **460.**

In figure 4E, a side detail view is provided of the stop cam **490.** In this view, a stop cam handle **491** is shown which allows the operator of the implant outside profile generator to fix the stop cam **490** in a first position A, and a second position B, whereby additional travel of the stop screw **470**, and thereby advancement of the carriage **442,** is provided in position **B**, of about 0.06" due to the difference in the distances shown for these positions.

By means of the apparatuses and method described above, a cortical bone implant **100** as shown in figure 1 having a substantially "D"-shaped external profile, and a substantially "D"-shaped internal canal is produced. Naturally, based on this disclosure, those skilled in the art will appreciate that other shapes, both for the external profile and internal canal of the implant may be produced. For example, an ellipsoid is produced by the above described methods simply by modification of the shape of the asymmetrically shaped cam **430**, and the internal canal shape may be modified by drilling, routing, or broaching using a broach that tapers to any desired shape. The thus formed implant may be used after machining as described, followed by appropriate cleaning methods known in the art (e.g. bathing in alcohol, peroxide treatment etc.). In addition, however, it may be desirable to inscribe an external feature on the upper surface **110**, the lower surface **111**, or both. Such a feature may take any desirable form, such as annular rings, indentations, projections, ribbing or teeth. In a preferred embodiment, teeth sloping toward the anterior aspect **108** of the implant are inscribed onto the top **110** and bottom **111** surfaces of the implant by forcing the implant through opposed broaches bearing inscribing teeth. Alternatively, the upper **110,** lower **111** or both surfaces in turn may be repeatedly run, manually or by a machine-driven means, over an appropriately fashioned jaw bearing abrasive teeth such that the required profile of teeth are inscribed into the surfaces of the implant. Desirably, the successive teeth of the jaw are incrementally raised in height such that each tooth is only required to remove a small amount of bone (about 0.004" per tooth, to a total depth of 0.015"). In addition, it is preferred that the rake (angle of the teeth) be sufficiently sharp as to allow the implant to bite into the implantation site, without at the same time being so sharp as to be excessively brittle.

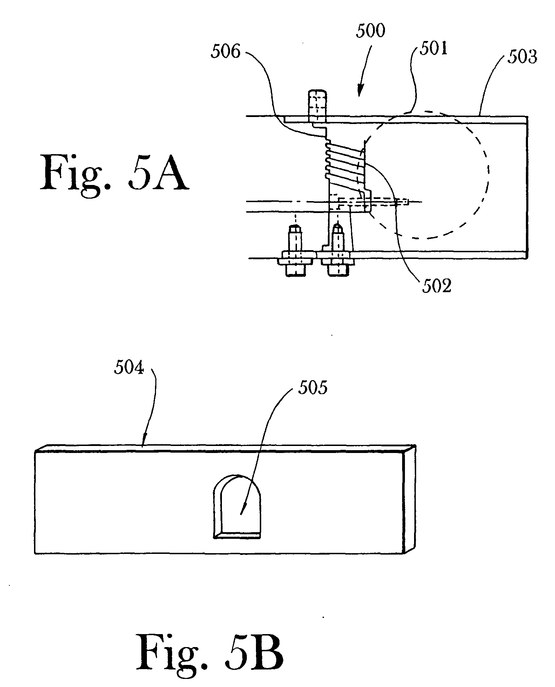

In figure 5, figure 5A, there is provided a top view of one side of one embodiment of blades **502** for use in a broach assembly **500** for inscribing teeth into the top **110,** bottom **111** or both surfaces of the implant. In outline, there is shown a lock-down handle 501 for clamping the assembly of blades **502** to a base **503**. By bringing a mirror image jaw into register with the depicted broach, a space is formed between the opposing teeth **502** at a distance sufficient to accommodate passage of the implant therebetween, provided that the teeth abrade recesses into the top and bottom surfaces of the implant **100**. To ensure proper engagement of the blades **502** and the implant **100**, there is provided a non-cutting surface 506 for contacting the implant 100 as it is introduced into the broach assembly **500**. The non-cutting surface **506** acts as a type of micrometer, forcing the cutting surfaces of the teeth **502** sufficiently apart to properly engage the implant as it passes through the broach assembly **500.** In figure 5B, there is provided a side view of an implant mounting device **504** having a "D"-shaped cavity **505** into which a "D"-shaped implant may be fitted for passage through the opposing jaws of the broaching jaw apparatus **500**. The resultant implant has the profile shown in figures 1C-1E.

In figures 5C-5E, there is shown an alternate apparatus and method for fashioning the retention teeth in the implant. In figure 5C, there is shown a carriage **510** having an appropriately dimensioned slot **520** for receiving the implant to be grooved. A tensioning screw **530** brings a retention arm **531** into juxtaposition with carriage housing member **532,** thereby clamping the implant into position within slot **520.** Through carriage housing members **532** and **533,** there is aligned a guide-rod **534** for guiding the carriage containing the implant as it is raked across a blade assembly **540,** over which said carriage **510** is made to pass. Said guide rod **540** also conveniently acts as a hinge, allowing the carriage **510** to swing upward for implant loading and also permitting the carriage to move down toward the base as the implant surface is cut on each successive pass of the carriage over said blade assembly **540.** The blade assembly **540** is bolted within a base **550** over which said carriage **510** slides. Said base **550** also acts to receive fixation screws **551** and **552** which retain said guide rod **534** in place. A plurality of individual blades **560** are placed in a recess **554** in the base **550** and are maintained in registered position by retention screws **552** passing through retention holes **553** in each blade. Each blade **560** has an initial non-cutting surface **561,** which is approximately 0.015" below the cutting surface **562,** which in combination with said plurality of blades, forms a flat loading area for implant insertion into said slot **520.** Figure 5D provides a side view of one blade **560,** while figure 5E provides an end on view of the carriage **510** as it sits above the base **550.** Accordingly, the implant is inserted into the slot **520** with the carriage **510** swung up from the base **550.** The carriage is then swung down into the starting position with the implant making contact with the non-cutting surfaces **561** of the plurality of blades. The implant is depressed so that it is forced snugly against the non-cutting surface, and then tensioned into place with the retention screw **530**. Thereafter, the carriage is slid several times over the base **550** such that the cutting surfaces **562** of the plurality of blades thereby inscribe the desired tooth structure into the top surface, the bottom surfaces or both (after switching the implant around) surfaces of the implant. When the metallic bottom of the carriage comes into contact with the base, the machining of the implant is complete.

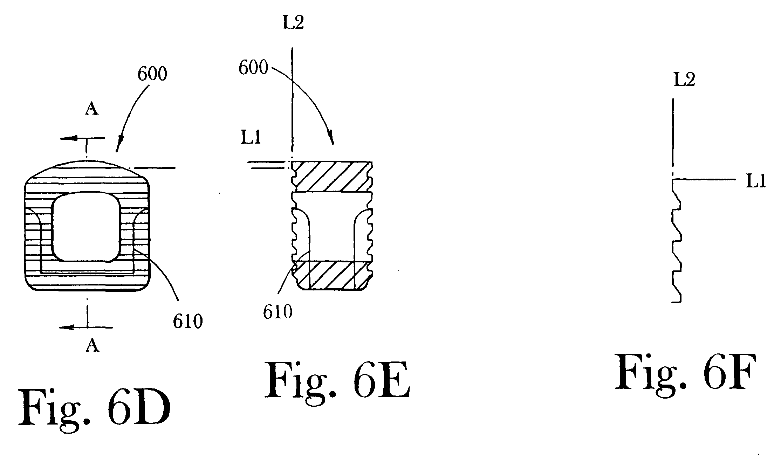

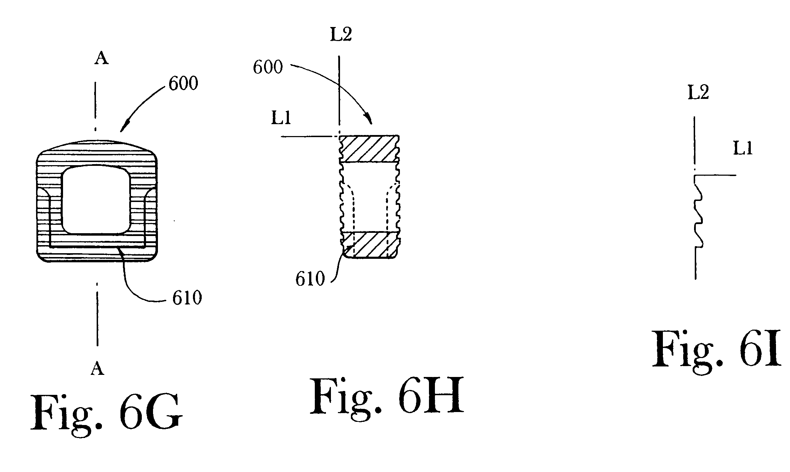

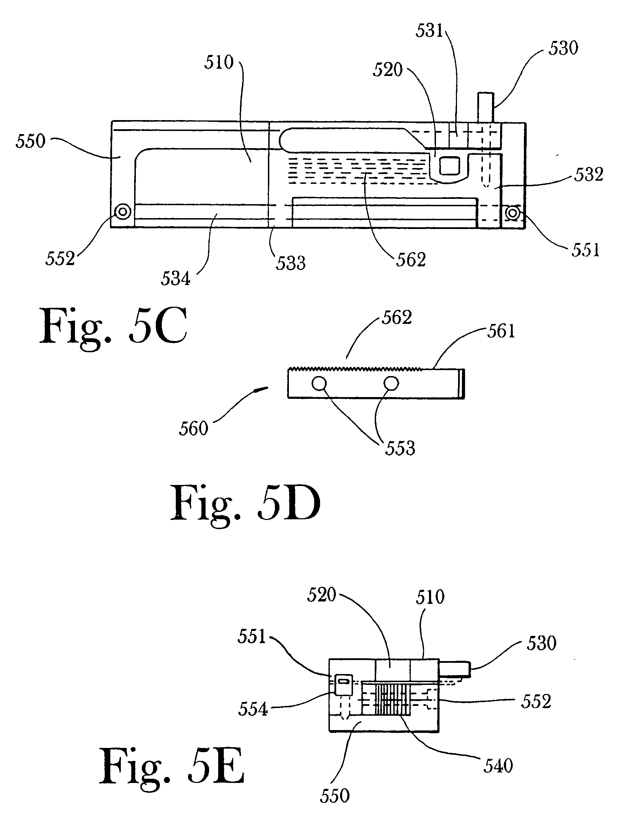

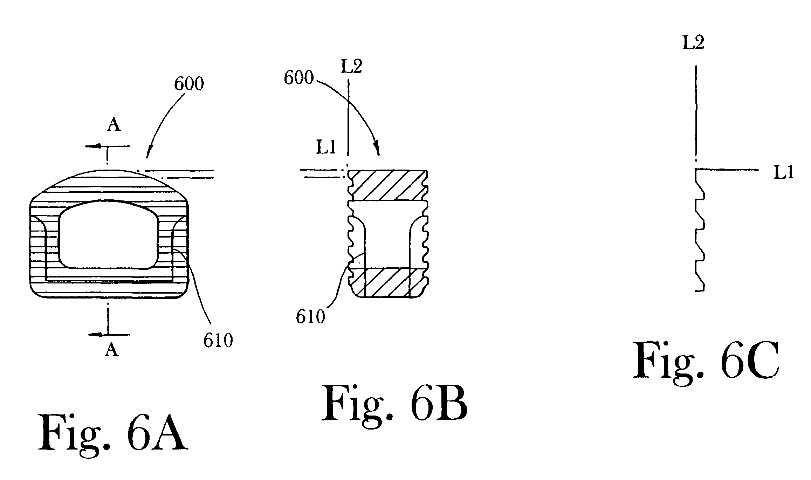

In figure 6A-I, there is provided a view of three different cortical bone implants according to this invention having particular geometries by way of example and not limitation. In figure 6A, there is shown an example of an implant **600** which has a height of 7 mm, a width of 11 mm, and a length of 14 mm. In addition, dimensions of various radii of the implant are provided. Note the effect of the "shoulder" **405** of the cutter which produces the a 0.059" radius and indent profile **610** starting at the approximate center of the part and proceeding around to the opposite side of the implant, i.e. around three faces of the implant. In figure 6B, the implant **600** is shown as a side view, and in figure 6C, there is shown a detail view of the teeth. Identical descriptions apply to the 7mmX 11 mmx 11mm views of the implants of figures 6D-6F and the 7mmX 14mmx 14mm implant of figures 6G-6I.

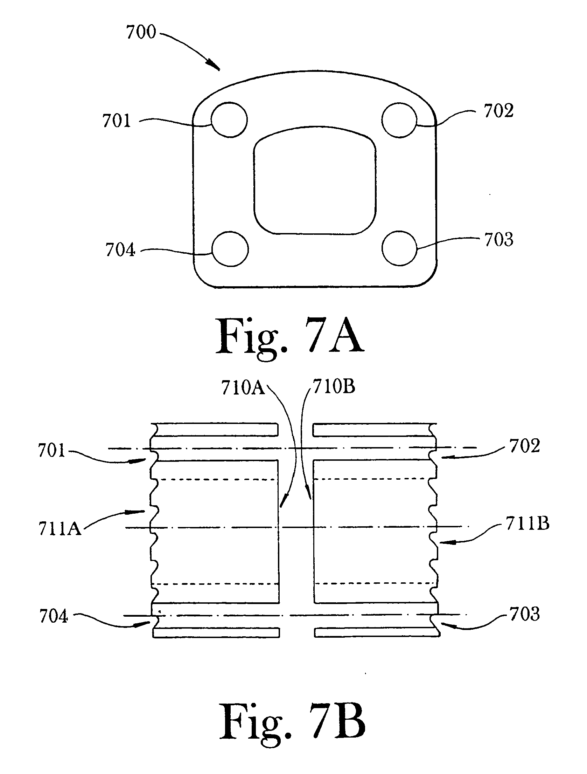

In figure 7, there is shown a further aspect of this invention in which an implant, either machined as described above, or prior to said machining, is further machined so as to allow stacking thereof to achieve implants of various heights. Commencing from a blank cortical plug at the stage shown in figure 2D has the advantage that if breakage of the implant occurs during machining, this will likely occur prior to completion of all of machining steps. According to this embodiment of the invention, two implant blanks of known height are selected such that a unitary implant composed of both starting implants can be produced of a new desired height (e.g. a 6 mm high implant may be stacked with a 7 mm high implant to produce a 13 mm implant). Each implant blank is placed in a drill jig, and by means of a drill press or like means, holes are drilled through the implants. With the implants still in the jig, the jig is placed on the table of an arbor press. Pins, composed of cortical bone, resorbable but strong biocompatible synthetic material, or metallic pins of the appropriate diameter are then impelled into the holes in the implants such that the implants are formed into a unitary body by these pins. In order to encourage bony ingrowth, channels may be cut into the adjacent surfaces of the implants. The embodiment shown in figure 7A is a top view of an implant **700** into which four holes **701-704** have been drilled. In figure 7B, there is shown the juxtaposition of two implants **700A** and **700B,** with the drilled holes **701-704** in register to receive pins for maintaining the implants in register. In this view, the adjacent surfaces **710A** and **710B** have not been inscribed with teeth, while the surfaces **711A** and **711B** have been so inscribed. Based on this disclosure, those skilled in the art will recognize that a number of variations and modifications may be made to stack various forms of bone implants, or to maintain such implants in register with each other. These modifications are to be considered within the scope of this invention. Thus, as shown in figure 9, an implant **900** is produced by producing two implants **901** and **902,** each having a cavity or canal 903, including an asymmetric key way **904** machined therein. By press-fitting the two implants together using an appropriately shaped cancellous plug **905** or a plug made from another biocompatible material, including but not limited to hydroxyapatite, cortical bone, synthetic materials, ceramic, optionally treated with growth factors such as bone morphogenetic protein and the like, the two implants **901** and **902** are retained in registered juxtaposition to form the implant **900.**

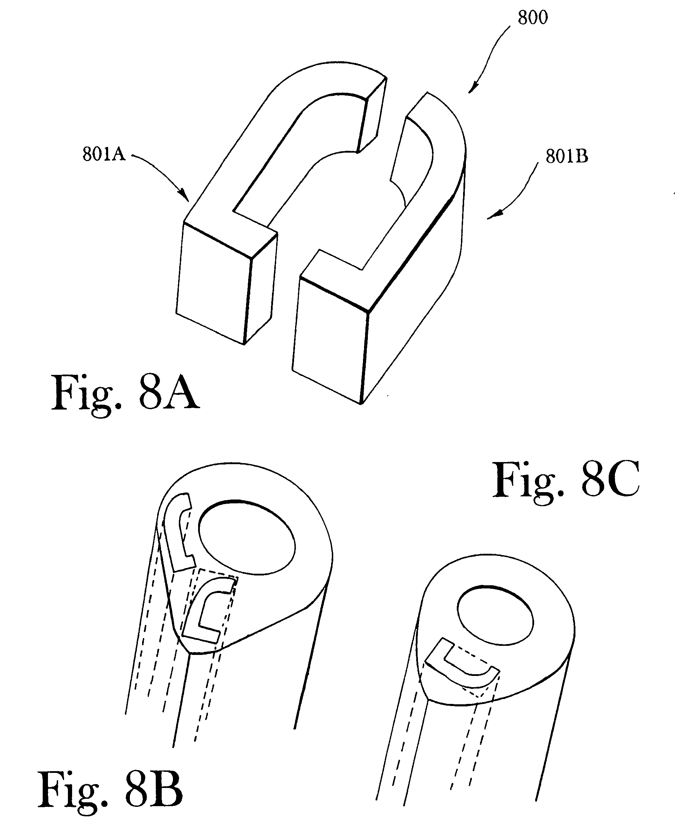

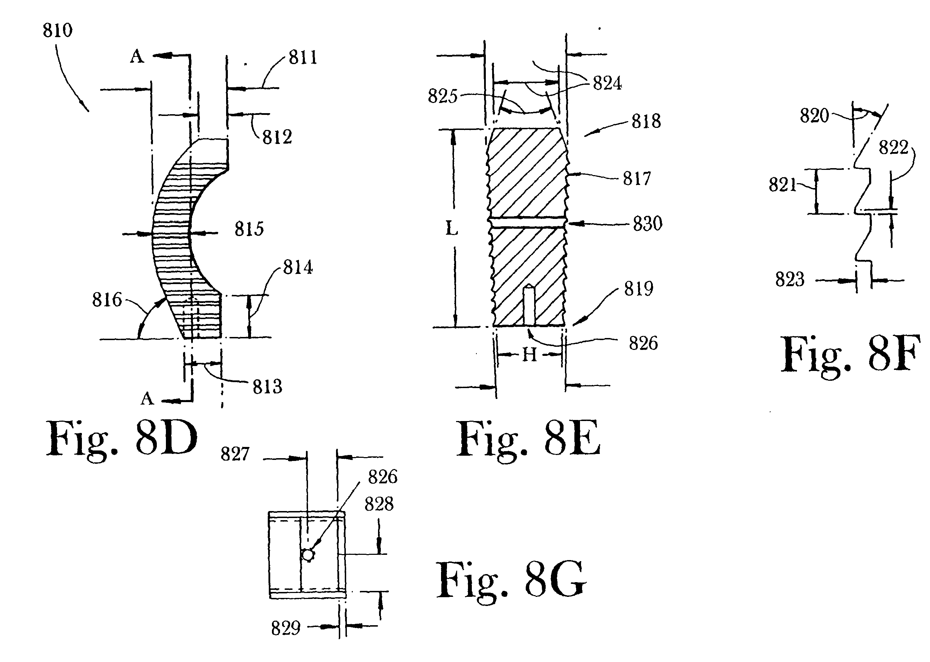

In a further embodiment of this invention, shown in figure 8, a method for assembling the implant of this invention from component parts is provided. In figure 8A, there is shown an implant **800** composed of two side-by-side halves, **801A** and **801B.** The two halves of the implant are brought into juxtaposition to form a unitary implant. The two halves may be implanted in juxtaposition, or holes may be formed in each half, and the halves maintained in contact by forcing pins through the holes, in a fashion analogous to that described above for maintaining stacked implants in contact with each other. For this embodiment of the invention, a portion of cortical bone may be harvested from any suitable source of cortical bone. As shown in figure 8B, a segment, in the form of a block or a column of cortical bone is harvested along the long axis of a long bone, such as the femur, tibia, or fibula. The shape of the bone may be inscribed into the thus-harvested cortical bone by routing, broaching or other means as described herein. The thus-machined cortical bone may then be sectioned into appropriate heights, as needed, to provide the implant halves **801A** and **801B.** Alternate sites for harvesting the cortical bone segment are shown in figures 8B and 8C.

In addition to use for cervical Smith-Robinson type fusion, implants comprising each element, **801A** or **801B** alone, modifications and variations thereof, optionally in combination with another vertebral fusion implant, may be implanted, for example, to assist in induction of posterior lumbar intervertebral fusion (PLIF). In such a case, a device **810,** such as that shown in figure 8D-8G is machined from bone stock as shown in figures 8B, 8C or another appropriate bone stock, and is inserted, according to methods known in the art for insertion of PLIF implants. Preferably, the device as used for PLIF applications has the following dimensions similar to the following, see side view figure 8D:a width **811** of approximately 7 to 12 mm, and preferably about 9.4 to about 10 mm; a top dimension **812** of about 4 to 5 mm; a bottom dimension **813** of about 4-6 mm and preferably about 5 mm; a flat surface of **814** of about 4-7 mm, and preferably about 5.5 mm; a width 815 of about 5-7 mm and preferably about 5 mm; a curvature that defines an angle 816 of between about 60 and 75 degrees, and preferably about 67 degrees. See figure 8E, rear view: a length L of about 20 to 26 mm; a height **H** of between about 7.5 and 14.5 mm; preferably, heights of about 8, 10, 12, and 14 mm are produced with lengths of about 20 and 26 mm; desirably, the side faces **817** are machined to display a rough, ridged or grooved surface so that when the anterior end **818** of the PLIF implant is properly seated in place, ridges directed to the posterior end **819** of the PLIF implant prevent backing out of the implant. A detail of one embodiment of such a ridged surface is shown in figure 8F, wherein the following dimensions are preferred: an angle **820** for each tooth of between about 30 and 40 degrees, preferably about 35 degrees; a distance between tooth crests **821** of about 1-2 mm, preferably about 1.5 mm; a tooth crest width **822** of about 0.1 to about 0.2 mm, preferably about 0.125 mm; and a tooth height **823** of between about 0.1 to about 1 mm and preferably about 0.5 mm; returning to figure 8E, the implant preferably has an anterior end width **824** of about 7-13 mm, preferably about 9-13 min, with a taper angle **825** from the height H of about 30 to 40 degrees, preferably about 35 degrees; an instrument attachment means, **826,** such as a tapped instrument attachment hole, is provided in the posterior face of the PLIF implant; this feature is best seen from the posterior view 8G, which shows: an instrument attachment hole **826** having a diameter of about 1.5 to about 2.5 mm, preferably about 2 mm, and a depth of about 4-5 mm, preferably about 4.5 mm; an edge to center of the instrument attachment hole dimension **827** is carefully defined to match dimensions of any implant insertion device used in combination with this embodiment of the PLIF implant; a center of the instrument attachment hole to edge dimension **828** is about 4-6 mm, preferably about 5 mm, with a ridge **829** of about I mm running along three edges of the posterior face of the implant. In displaying the section A-A from figure 8D in figure 8E, a slight air gap **830** is shown as the section would exit bone on the concave surface of the implant and then reenter bone.

In use, the implant **810** is inserted on either side of lumbar intervertebral spaces to thereby stabilize and assist in fusion of adjacent lumbar vertebrae. The convex surface of the implant **810** is set to match, as much as possible, the natural external curvature of the lumbar vertebrae.

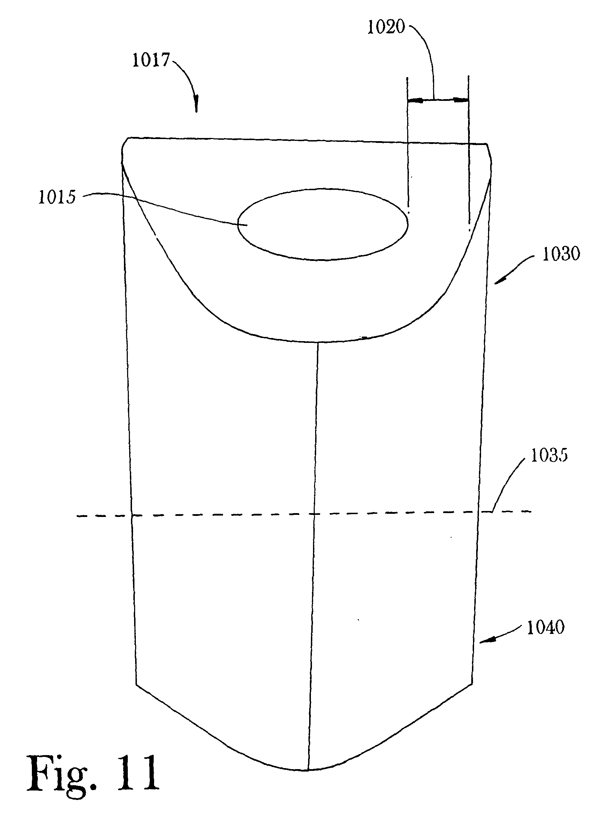

In an analogous but alternate method for production of the cervical implant, unitary implants may be produced by appropriately sectioning and machining the anterior margin of the tibia or linea aspera of the femur. Thus, as shown in figure 10A, a left femur **1000** (posterior aspect), or in figure 10B, a left tibia **1001** (anterior aspect), is sectioned at **1004** and **1005** to remove the head, neck and greater trochanter **1002** and internal and internal condyles **1006** of the femur, or tubercle and tuberosity **1003** malleolus **1007** of the tibia. The result from such sectioning is the production of a shaft, or diaphysis, of the femur **1008** or tibia **1009.** Further processing according to this aspect of the invention involves the line asper **1010** of the femur or the anterior margin of the tibia **1011,** as shown in figure 10C. Whether produced from the femur or tibia, a diaphysial shaft **1012,** extending as shown at **1016** to a length permitted by the length of the shaft produced by the sectioning at **1004/1005.** The shaft comprises the natural intramedullary canal **1013.** The thus produced shaft is then further sectioned in a plane shown at **1014** to produce a shaft of bone removed from the natural intramedullary canal **1013** having a cylindrical but somewhat triangular external shape. Into this shaft may be drilled a cannulation **1015,** as shown in figure 11.

Figure 11 shows the substantially triangular shaft of substantially cortical bone **1017** produced by sectioning the shaft of the long bone down the plane **1014.** Into the shaft of bone **1017** may be drilled a bore to produce a cannulation 1015 of appropriate dimensions. The cannulation **1015** may be introduced into the unitary shaft of bone **1017** or it may be introduced into sub-segments thereof by first cutting the shaft **1017** at **1035.** In either case, the diameter of the cannulation **1015** should be limited such that at the narrowest portion between the cannulation and the wall of the device **1020** never falls below about 2 mm. When sectioned at **1035,** for example by mounting the shaft **1017** on a lathe and contacting the spinning shaft with a very narrow blade (i.e. a parting tool of about 1 mm or less width), implant blanks **1030** and **1040** are produced which may be further machined to achieve desired external and internal profiles and key way features, as described for the implant of this invention produced by alternate methods described hereinabove. Implants of any desired height, for example 5 mm to about 14 mm, may thus be produced. Figures 12-17 show specific embodiments of the implant of this invention produced according to this aspect of the invention.

Per figures 12-17, there is provided views of five different cortical bone implants according to this invention having particular geometries by way of example and not limitation. In each figure, view A is a top view, view B is a side view, view C is a detail of the grooves which angle toward the posterior aspect of the implant, and view D is a sectional view through the line A-A shown in view A. In addition, where an osteogenic plug, such as a cancellous plug is present, this is shown in view E as a top view and view F as a side view of the cancellous plug. In figure 12, there is shown an example of an implant having a height **H1** between about 5mm and about 9mm, a width **W1** of about 11 mm, and a width **W2** of about 11 mm. An outer dotted profile provides a means for comparing the shape of the implant produced according to this alternate manufacturing method with the external profile of the implant of figure 6. As can be seen, the implant produced according to this aspect of the invention has a substantially diamond-shaped external profile, as a result of the geometry of the starting shaft **1017** of bone stock.

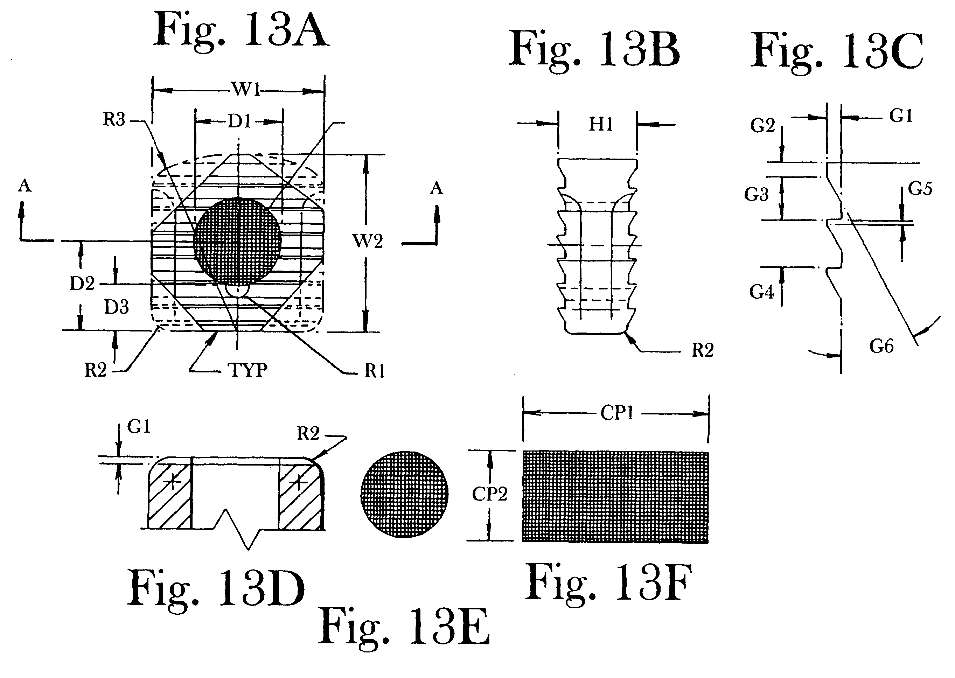

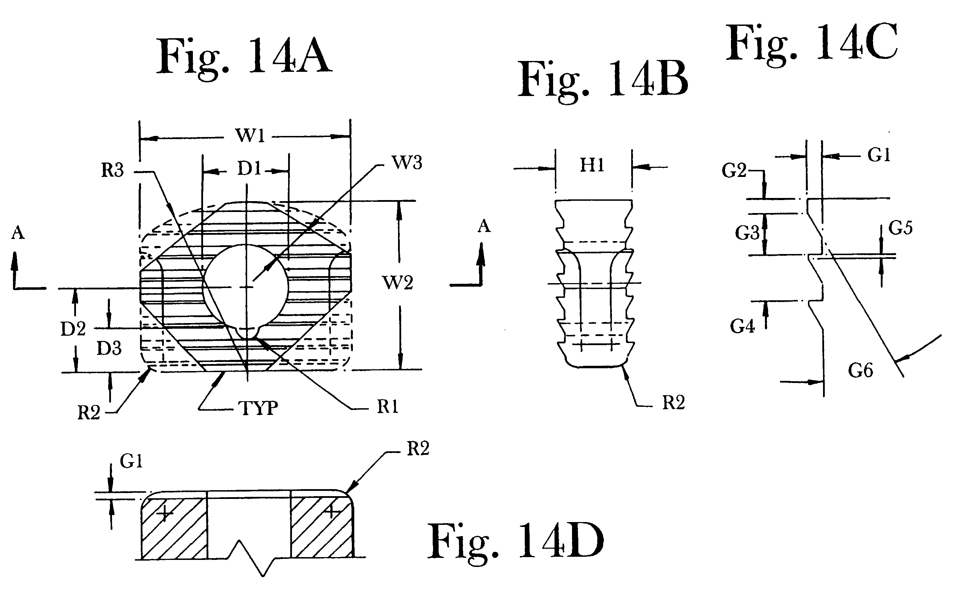

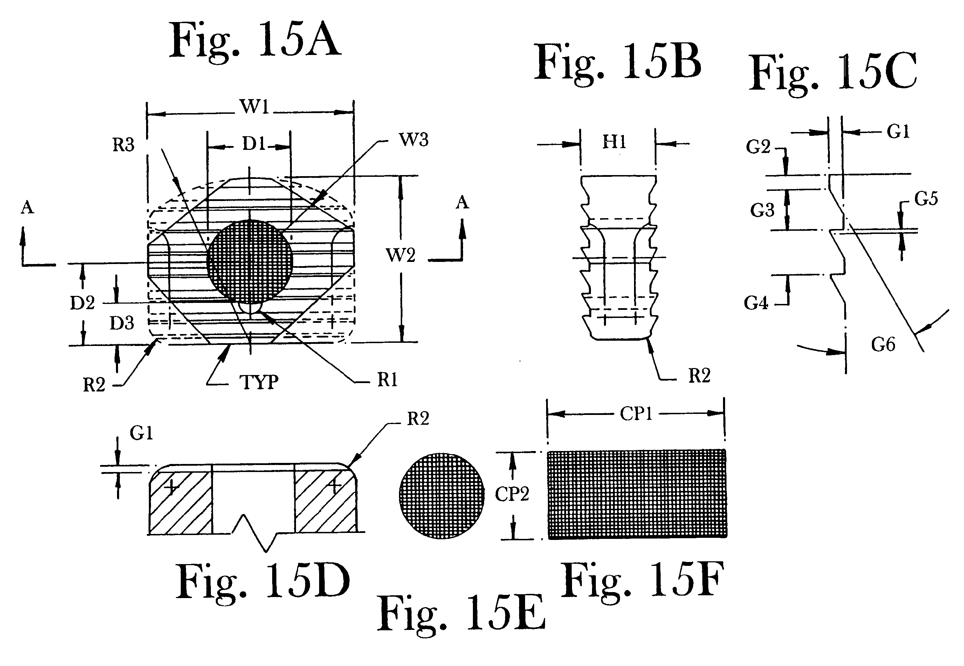

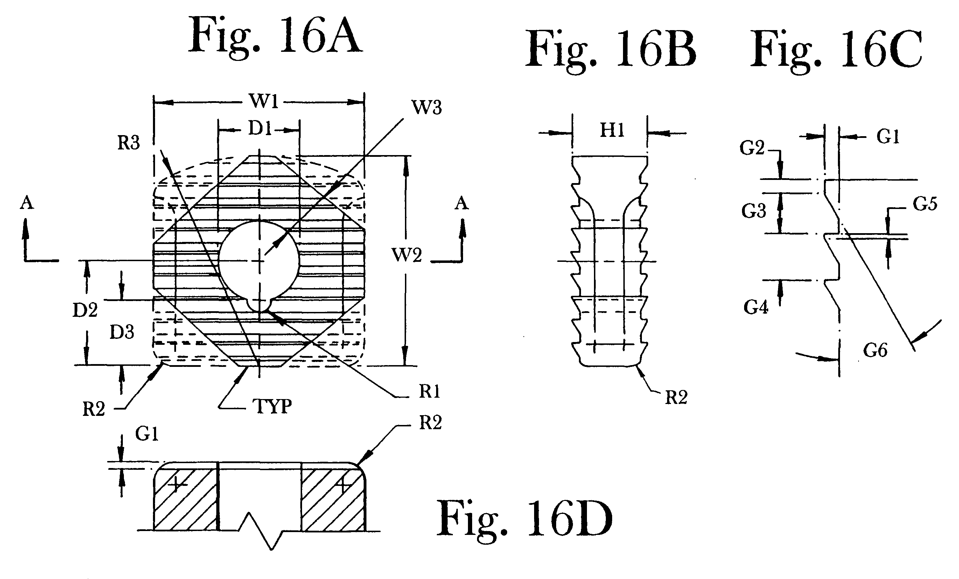

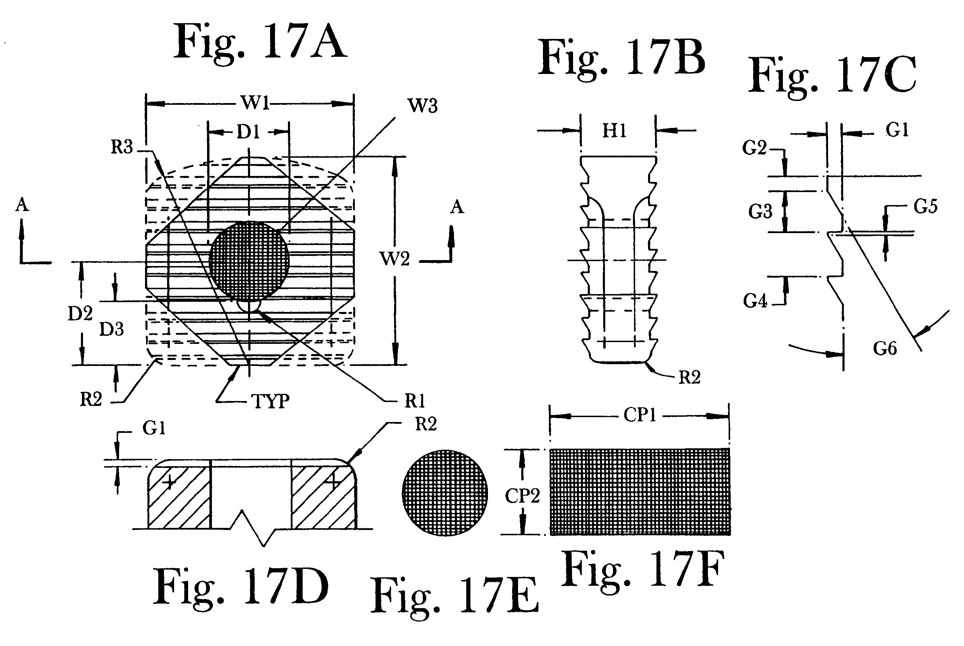

Figure 13 shows a device similar to that of figure 12, with a cancellous plug inserted therein. Figure 14 shows a device having a width **W1** of about 14 mm and a height **H1** of between about 5 mm and about 14 mm. Figure 15 shows a device similar to that of figure 14 with a cancellous plug inserted therein. Figure 16 shows a device having a width **W1** of about 14 mm, a width **W2** of about 14 mm, and a height of between about 5 mm and 11 mm. Figure 17 shows a device similar to that of figure 16 having a cancellous plug inserted therein. Table I below summarizes the various features and provides examples of specific dimensions for various embodiments of the implant of this invention shown in figures 12-17:

**Table I:**

| Figure # | | | | | | | |
|---|---|---|---|---|---|---|---|
| Code | Description | 12 | 13 | 14 | 15 | 16 | 17 |
| D1 | Inner Hole Diameter | 5.5 | 5.5 | 5.5 | 5.5 | 5.5 | 5.5 |
| D2 | Hole Centerline Distance | 5.5 | 5.5 | 5.5 | 5.5 | 5.5 | 5.5 |
| D3 | Key Way Centerline Distance | 2.9 | 2.9 | 2.9 | 2.9 | 4.4 | 4.4 |
| G1 | Groove Depth | 0.5 | 0.5 | 0.5 | 0.5 | 0.5 | 0.5 |
| G2 | Top Flat Width | 0.5 | 0.5 | 0.5 | 0.5 | 0.5 | 0.5 |
| G3 | Groove Pitch width without G5 | 1.375 | 1.375 | 1.375 | 1.375 | 1.375 | 1.375 |
| G4 | Groove Pitch width with G5 | 1.5 | 1.5 | 1.5 | 1.5 | 1.5 | 1.5 |
| G5 | Flat Width | 0.125 | 0.125 | 0.125 | 0.125 | 0.125 | 0.125 |
| G6 | Groove Angle (degrees) | 30 | 30 | 30 | 30 | 30 | 30 |
| H1 | Overall Height | 5-9 | 5-9 | 5-13 | 5-13 | 5-11 | 5-11 |
| R1 | Keyway Radius | 0.8 | 0.8 | 0.8 | 0.8 | 0.8 | 0.8 |
| R2 | Corner Radius | 1.5 | 1.5 | 1.5 | 1.5 | 1.5 | 1.5 |
| R3 | Outside Arch Radius | 11 | 11 | 11 | 11 | 14 | 14 |
| W1 | Outside Width | 11 | 11 | 14 | 14 | 14 | 14 |
| W2 | Outside Width | 11 | 11 | 11 | 11 | 14 | 14 |
| W3 | Wall Thickness | ≥2 | ≥2 | ≥2 | ≥2 | ≥2 | ≥2 |
| CP1 | Cancellous Plug Height | N/A | 5-9 | N/A | 5-13 | N/A | 5-11 |
| CP2 | Cancellous Plug Diameter | N/A | 5.7-5.8 | N/A | 5.7-5.8 | N/A | 5.7-5.8 |

### 4.4 Manner of Using the Implant

In use, the implant **100** is inserted into a space formed between adjacent vertebrae that are required to be fused. This may be accomplished by the surgeon removing portions of the intervertebral disk, (partial discectomy) and retracting the adjacent vertebrae to allow insertion of an appropriately dimensioned implant. The rear end **105** of the implant is inserted first, and where present, the external feature **120** prevents backing out of the implant. Where no external feature **120** has been inscribed into the top and bottom surfaces of the implant, it may be necessary to affix the implant in position with plate and screw retention systems known in the art. According to this invention, implants are provided having a height of between about 7 and 14 mm, a length of between about 11 and 14 mm and a width of between about 11 and 14 mm. Any permutation or combination of these dimensions may be envisioned, for example (in order of height, length, width): 7x11x11, 8x11x11, etc.; 7x14x14, 8x14x14, etc.; 7x11x14, 8x11x14, etc.

Preferably, the surgeon performing the implantation saves the autologous material and debris produced in the course of the partial discectomy for packing into the canal of the present implant. In addition, or alternatively, the canal may be packed (either during the surgical procedure or the canal may be pre-packed) with osteogenic, osteoinductive, or osteoconductive materials, including but not limited to: allograft bone, autograft bone, autogenous osteogenic materials including bone marrow cancellous bone and the like, demineralized bone, freeze-dried demineralized bone, Grafton® (demineralized bone in glycerol), bone powder, bone derivatives, bone morphogenetic protein (purified or recombinant), antibiotic, bioactive glass, hyrdorxyapatite, bioactive ceramics, or combinations thereof.

Following implantation, the recipient (whether human or animal) is monitored for implant stability and success in fusion. Fusion is achieved over the course of several weeks to several months, during which time increasing levels of load may be placed on the spine.

### 5.0 References

Bailey, R.W., and Badgley, L.E. (1960) *Stabilization of the Cervical Spine by Anterior Fusion,* J. Bone and Joint Surg. **42A**:565-594.
Cloward, R. B. (1958) *The Anterior Approach for Removal of Ruptured Cervical Discs,* J. Bone and Joint Surg. **15**:602-617.
Grooms *et al.,* (1996) *Success of Surgery on the Anterior Cervical Spine: Smith-Robinson Technique vs. Internal Plates,* Clinical Performance of Skeletal Prostheses, L.L. Hench and J. Wilson, Eds., Chapman & Hall.
Robinson, R.A., *et al.,* (1962) *The Results ofAnterior Interbody Fusion of the Cervical Spine, J.* Bone and Joint Surg. **44A**:1569-1587.
Robinson, R.A. and Smith, G.W. (1955) *Anterolateral Cervical Disc Removal and Interbody Fusion for Cervical Disc Syndrome,* Bull. Johns Hopkins Hosp. **96**:223-224.
Smith, G.W. and Robinson, R.A., (1958), *The Treatment of Certain Cervical-Spine Disorders by Anterior Removal of the Intervertebral Disc and Interbody Fusion,* J. Bone and Joint Surg. **40A**:607-623.
White, A.A. III, and Hirsh, C. (1972) *An Experimental Study of the Immediate Load Bearing Capacity of Some Commonly Used Iliac Bone Grafts,* Acta Orthop. Scandanav. **42:**482-490.
Whitecloud, T.S. III and Dunsker, S. (1993) *Anterior Cervical Spine Surgery, Principles and Techniques in Spine Surgery,* Raven Press, N.Y.
U.S. Patent No. 5,306,309
U.S. Patent No. 5,609,635
U.S. Patent No. 5,306,307
U.S. Patent No. 4,950,296

## Claims

1. A substantially D-shaped implant consisting substantially of cortical bone comprising a convexly curved anterior cortical bone face 108 and three rectilinear cortical bone faces 105 unitary with said convexly curved anterior bone face, and a canal 104 surrounded by a machined wall of cortical bone in the shape of a circle, ellipse, or an asymmetric shape, including a substantially D-shape, thereby forming an implant having a top face and a bottom face, said top face or bottom face or both having a series of teeth 110 which angle toward said convexly curved anterior bone face 108.

2. The implant of claim 1, wherein said canal has a substantially "D"-shape.

3. The implant of claim 1 or claim 2, wherein an osteogenic, osteoinductive or osteoconductive composition is packed within said canal.

4. The implant of claim 3, wherein said osteogenic, osteoinductive or osteoconductive composition derives from the intervertebral space into which said implant is inserted, is hydroxyapatite, bone powder, bone product, bone morphogenetic protein, bioactive glass, bioactive ceramic, or combinations of these.

5. The implant of any preceding claim comprising discontinuous walls consisting substantially of cortical bone, wherein said discontinuous walls are mirror image halves which, in combination, form said shape.

6. The implant according to claim 5, wherein said mirror image halves have a substantially half-D shape with a width, top dimension, bottom dimension, a curvature defining an inner, concave surface and an outer, convex surface, a length and a height adapted for posterior lumbar intervertebral fusion.

7. The implant according to claim 6, wherein said implant is shaped to be inserted on either side of lumbar intervertebral spaces to thereby stabilise and assist in fusion of adjacent lumbar vertebrae and wherein said convex surface is set to match, as much as possible, the natural external curvature of the lumbar vertebrae.

8. The implant of claim 1, in stacked combination with one or more second implant (s) of claim 1, optionally pinned to each other.

9. The implant of any of claims 1 to 7, in combination and juxtaposition to a second implant of any of claims 1 to 7.

10. The combination of claim 9, being held together by one or more cortical bone pins.

11. The implant of claim 9, wherein said combination of implants is adapted to form a unitary implant for implantation into an appropriately dimensioned cavity formed between adjacent vertebrae.

12. The implant of claim 9 or claim 11, wherein the combined implants are pinned to each other by cortical bone pins, said pins consisting of biocompatible synthetic material or metallic pins.

13. The implant of any of claims 9 to 12, wherein the combined implants are two mirror image halves of a desired shape.

14. A method of making an implant according to claim 1, comprising:
(a) cutting a segment of cortical bone
(b) machining the segment of cortical bone of step (a) to produce a "washer-shaped" segment;
(c) machining a canal through the washer shaped segment to form an asymmetric shape therein; and
(d) using said asymmetric shape to machine an outside profile of the bone segment.

15. A method for making the implant of claim 5, comprising:
(a) cutting a segment of cortical bone
(b) shaping a segment of cortical bone into a mirror image half of the final shape of said implant comprising a canal surrounded by a continuous or discontinuous wall of cortical bone, such that when implanted in juxtaposition with an opposing mirror image half, an implant is formed having a D-shape, a top face and a bottom face, each of which face is substantially planar, with said planes being substantially parallel to each other, and
(c) cutting appropriate lengths of said shaped segment of cortical bone such that said cut length provides said mirror image half of an implant having a desired height.

16. The method of claim 14, wherein said segment of bone is obtained by cutting into the diaphysis of a long bone and into the intramedullary canal of said long bone to form a bone segment having a substantially cortical end and an end derived from the wall of the intramedullary canal.

17. The method of claim 16, wherein the end of the segment of bone derived from the intramedullary canal is machined to form a substantially planar surface to obtain a substantially "washer-shaped" bone segment composed substantially of cortical bone.

18. The method of claim 17, wherein said canal is formed into an asymmetric shape by broaching said canal to form said asymmetric shape through the bone segment.

19. The method of claim 18, wherein said asymmetric shape is substantially "D"-shaped.

20. The method of claim 18, wherein said bone segment having a substantially "D"-shaped canal is further machined such that the external profile of the bone segment substantially matches the profile of said canal.

21. The method of claim 18, wherein said further machining comprising contacting the bone segment with an asymmetrically shaped grinder wheel.

22. The method of claim 18, wherein said further machining comprises mounting said bone segment on a spindle affixed to an asymmetrically shaped cam and containing the thus mounted bone segment with a cutter rotating about a symmetric axis such that the cutter is made to cut more or less bone as dictated by the shape of said asymmetric cam.

23. The method of claim 22, further comprising stacking said bone segment, either prior to or after said machining, drilling holes therein, and pinning said stacked bone segments to each other.

24. The method of claim 14, which further comprises machining an external feature into the top the bottom or both surfaces of the implant.

25. The method of claim 24, wherein said external feature is machined by passing said implant through a broach or by repeatedly passing said implant over a plurality of cutting teeth.

## Patentansprüche

1. Ein im Wesentlichen D-förmiges, im Wesentlichen aus kortikalem Knochen bestehendes Implantat, das eine konvex gekrümmte vordere kortikale Knochenstirnfläche 108 und drei geradlinige, mit der konvex gekrümmten vorderen kortikalen Knochenstirnfläche einheitliche Knochenstirnflächen 105 und einen Kanal 104 umfasst, der von einer maschinell bearbeiteten Wand aus kortikalem Knochen in der Form eines Kreises, einer Ellipse oder einer asymmetrischen Form einschließlich einer im Wesentlichen D-Form umgeben ist, wodurch ein Implantat mit einer oberen Stirnfläche und einer unteren Stirnfläche gebildet ist, wobei die obere Stirnfläche oder untere Stirnfläche oder beide eine Reihe an Zähnen 110 aufweisen, die in Richtung auf die konvex gekrümmte vordere Knochenstirnfläche 108 zu gewinkelt sind.

2. Implantat nach Anspruch 1, bei dem der Kanal eine im Wesentlichen "D"-Form hat.

3. Implantat nach Anspruch 1 oder Anspruch 2, bei dem eine osteogene, osteoinduktive oder osteokonduktive Zusammensetzung in den Kanal gepackt ist.

4. Implantat nach Anspruch 3, bei dem die osteogene, osteoinduktive oder osteoconduktive Zusammensetzung aus dem Zwischenwirbelraum stammt, in den das Implantat eingesetzt wird, Hydroxyapatit, Knochenmehl, ein Knochenprodukt, ein knochenmorphogenetisches Protein, bioaktives Glas, bioaktive Keramik oder Kombinationen von diesen ist.

5. Implantat nach einem vorherigen Anspruch, mit diskontinuierlichen Wänden, die im Wesentlichen aus kortikalem Knochen bestehen, wobei die diskontinuierlichen Wände Spiegelbildhälften sind, die in Kombination die Form bilden.

6. Implantat nach Anspruch 5, bei dem die Spiegelbildhälften eine im Wesentlichen Halb-D-Form mit einer Breite, oberen Abmessung, unteren Abmessung, einer eine innere, konkave Oberfläche und eine äußere, konvexe Oberfläche definierende Krümmung, eine Länge und eine Höhe haben, die zur posterioren lumbalen Zwischenwirbelverbindung ausgelegt sind.

7. Implantat nach Anspruch 6, bei dem das Implantat geformt ist, um an eine Seite von lumbalen Zwischenwirbelräumen eingesetzt zu werden, um **dadurch** eine Verbindung von benachbarten lumbalen Wirbeln zu stabilisieren und zu unterstützen, und bei dem die konvexe Oberfläche hergerichtet ist, um so gut wie möglich zu der natürlichen äußeren Krümmung der lumbalen Wirbel zu passen.

8. Implantat nach Anspruch 1 in gestapelter Kombination mit einem oder mehreren zweiten Implantat(en) nach Anspruch 1, die optional miteinander verstiftet sind.

9. Implantat nach einem der Ansprüche 1 bis 7, in Kombination und Nebeneinanderstellung mit einem zweiten Implantat nach einem der Ansprüche 1 bis 7.

10. Kombination nach Anspruch 9, die durch einen oder mehrere kortikale Knocheristifte zusammengehalten ist.

11. Implantat nach Anspruch 9, bei dem die Kombination von Implantaten ausgelegt ist, ein einheitliches Implantat zur Implantierung in eine geeignet dimensionierte, zwischen benachbarten Wirbeln gebildete Kavität zu bilden.

12. Implantat nach Anspruch 9 oder Anspruch 11, bei dem die kombinierten Implantate mittels kortikaler Knochenstifte miteinander verstiftet sind, wobei die Stifte aus biokompatiblem synthetischen Material oder metallischen Stiften bestehen.

13. Implantat nach einem der Ansprüche 9 bis 12, bei dem die kombinierten Implantate zwei Spiegelbildhälften einer gewünschten Form sind.

14. Verfahren zur Herstellung eines Implantats nach Anspruch 1, mit:
(a) Schneiden eines Segments aus kortikalem Knochen;
(b) maschinellem Bearbeiten des Segments aus kortikalem Knochen von Schritt (a), um ein "unterlegscheibenförmiges" Segment herzustellen;
(c) Fertigen eines Kanals durch das unterlegscheibenförmige Segment, um darin eine asymmetrische Form auszubilden; und
(d) Verwenden der asymmetrischen Form, um ein Außenseitenprofil des Knochensegments maschinell herzustellen.

15. Verfahren zur Herstellung des Implantats nach Anspruch 5, mit:
(a) Schneiden eines Segments aus kortikalem Knochen;
(b) Formen eines Segments aus kortikalem Knochen in eine Spiegelbildhälfte der endgültigen Form des Implantats mit einem Kanal, der von einer kontinuierlichen oder diskontinuierlichen Wand aus kortikalem Knochen umgeben ist, so dass, wenn in Nebeneinanderstellung mit einer gegenüber liegenden Spiegelbildhälfte implantiert, ein' Implantat gebildet wird, das eine D-Form, eine obere Stirnfläche und eine untere Stirnfläche aufweist, wobei jede der Stirnflächen im Wesentlichen planar ist, wobei die Ebenen im Wesentlichen parallel zueinander sind; und
(c) Schneiden geeigneter Längen des geformten Segments aus kortikalem Knochen, so dass die geschnittene Länge die Spiegelbildhälfte eines Implantats mit einer gewünschten Höhe bereitstellt.

16. Verfahren nach Anspruch 14, bei dem das Segment aus Knochen erhalten wird, indem in die Diaphyse eines langen Knochens und in den intramedullären Kanal des langen Knochens geschnitten wird, um ein Knochensegment mit einem im Wesentlichen kortikalen Ende und einem Ende zu bilden, das von der Wand des intramedullären Kanals stammt.

17. Verfahren nach Anspruch 16, bei dem das Ende des Segments aus Knochen, das aus dem intramedullären Kanal stammt, maschinell bearbeitet wird, um eine im Wesentlichen planare Oberfläche zu bilden, um ein im Wesentlichen "unterlegscheibenförmiges" Knochensegment zu erhalten, das im Wesentlichen aus kortikalem Knochen zusammengesetzt ist.

18. Verfahren nach Anspruch 17, bei dem der Kanal in eine asymmetrische Form ausgebildet wird, indem der Kanal ausgeräumt wird, um die asymmetrische Form durch das Knochensegment hindurch zu bilden.

19. Verfahren nach Anspruch 18, bei dem die asymmetrische Form im Wesentlichen "D"-förmig ist.

20. Verfahren nach Anspruch 18, bei dem das Knochensegment, das einen im Wesentlichen "D"-förmigen Kanal aufweist, weiter maschinell bearbeitet wird, so dass das äußere Profil des Knochensegments im Wesentlichen dem Profil des Kanals entspricht.

21. Verfahren nach Anspruch 18, bei dem das weitere maschinelle Bearbeiten umfasst, das Knochensegment mit einer asymmetrisch geformten Schleifscheibe zu kontaktieren.

22. Verfahren nach Anspruch 18, bei dem das weitere maschinelle Bearbeiten umfasst, das Knochensegment an einer Spindel zu befestigen, die an einem asymmetrisch geformten Mitnehmer angeordnet ist, und das so befestigte Knochensegment mit einem Schneidwerkzeug zu kontaktieren, das sich um eine symmetrische Achse dreht, so dass das Schneidwerkzeug, wie durch die Form des asymmetrischen Mitnehmers vorgegeben, mehr oder weniger Knochen schneidet.

23. Verfahren nach Anspruch 22, ferner umfassend, das Knochensegment entweder vor oder nach dem maschinellen Bearbeiten zu stapeln, Löcher hinein zu bohren und die gestapelten Knochensegmente miteinander zu verstiften.

24. Verfahren nach Anspruch 14, ferner umfassend, ein äußeres Merkmal in die obere, die untere oder beide Oberflächen des Implantats maschinell einzuarbeiten.

25. Verfahren nach Anspruch 24, bei dem das äußere Merkmal maschinell eingearbeitet wird, indem das Implantat durch eine Aale hindurch geführt wird oder indem das Implantat wiederholt über eine Mehrzahl von Schneidzähnen geführt wird.

## Revendications

1. Implant sensiblement en forme de D, consistant sensiblement en l'os cortical, comprenant une face d'os cortical antérieur 108, incurvé de manière convexe, et trois faces d'os cortical linéaire 105, unitaires avec ladite face d'os antérieure, incurvée de manière convexe, et un canal 104 entouré d'une paroi usinée d'os cortical en la forme d'un cercle, d'une ellipse ou en une forme asymétrique, comprenant sensiblement une forme de D, ce qui forme un implant ayant une face supérieure et une face inférieure, ladite face supérieure ou face inférieure ou les deux, ayant une série de dents 110, qui font un angle avec ladite face d'os antérieur 108, incurvée de manière convexe.

2. Implant selon la revendication 1, où ledit canal a sensiblement une forme de D.

3. Implant selon la revendication 1 ou 2, où une composition ostéogène, ostéoinductrice ou ostéoconductrice est disposée dans ledit canal.

4. Implant selon la revendication 3, où ladite composition ostéogène, ostéoinductrice ou ostéoconductrice dérive de l'espace intervertébral dans lequel ledit implant est inséré, est de l'hydroxyapatite, de la poudre d'os, un produit osseux, une protéine morphogénétique de l'os, un verre bioactif, une céramique bioactive ou des combinaisons de ceux-ci.

5. Implant selon l'une quelconque des revendications précédentes, comprenant des parois discontinues consistant sensiblement en l'os cortical, où lesdites parois discontinues sont des moitiés chirales, qui par combinaison, forment ladite forme.

6. Implant selon la revendication 5, où lesdites moitiés chirales ont sensiblement une forme de D, avec une largeur, une dimension supérieure, une dimension inférieure, une courbure définissant un intérieur, une surface concave et une surface convexe, extérieure, une longueur et une hauteur adaptés à une fusion intervertébrale lombaire postérieure.

7. Implant selon la revendication 6, où ledit implant a une forme telle qu'il puisse être inséré sur un côté des espaces intervertébraux lombaires pour ainsi stabiliser et aider à la fusion de vertèbres lombaires adjacentes et où ladite surface convexe est prévue pour s'accorder autant que possible à la courbure externe naturelle des vertèbres lombaires.

8. Implant selon la revendication 1, en combinaison empilée avec un ou plusieurs implants de la revendication 1, le cas échéant chevillés les uns aux autres.

9. Implant selon l'une quelconque des revendications 1 à 7, en combinaison et juxtaposition avec un deuxième implant selon l'une quelconque des revendications 1 à 7.

10. Combinaison selon la revendication 9, maintenue ensemble par une ou plusieurs broches d'os cortical.

11. Implant selon la revendication 9, dans lequel ladite combinaison des implants est adaptée pour former un implant unitaire pour une implantation dans une cavité de dimension appropriée, formée entre des vertèbres adjacentes.

12. Implant selon la revendication 9 ou 11, où les implants combinés sont chevillés l'un à l'autre par des broches d'os cortical, lesdites broches consistant en un matériau synthétique biocompatible ou sont des broches métalliques.

13. Implant selon l'une quelconque des revendications 9 à 12, où les implants combinés sont deux moitiés chirales d'une forme désirée.

14. Procédé de préparation d'un implant selon la revendication 1, comprenant
(a) la découpe d'un segment d'os cortical,
(b) l'usinage du segment d'os cortical de l'étape (a) pour produire un segment « en forme de rondelle »,
(c) l'usinage d'un canal au travers du segment en forme de rondelle pour y former une forme asymétrique, et
(d) l'utilisation de ladite forme asymétrique pour usiner un profil extérieure au segment d'os.

15. Procédé pour préparer l'implant de la revendication 5, comprenant
(a) la découpe d'un segment d'os cortical,
(b) la mise en forme du segment d'os cortical en une moitié chirale de la forme finale dudit implant, comprenant un canal entouré d'une paroi continue ou discontinue d'os cortical, de sorte que lorsqu'il est implanté en juxtaposition avec une moitié chirale opposée, on forme un implant en forme de D, avec une face supérieure et une face inférieure, chacune des face étant sensiblement plane, lesdits plans étant sensiblement parallèles l'un à l'autre, et
(c) la découpe de longueurs appropriées dudit segment mis en forme d'os cortical, de sorte que ladite longueur coupée donne une moitié chirale d'un implant ayant une hauteur souhaitée.

16. Procédé selon la revendication 14, dans lequel ledit segment d'os est obtenu par découpe dans la diaphyse d'un os long et dans le canal intramédullaire dudit os long pour former un segment d'os ayant une extrémité sensiblement corticale et une extrémité dérivée de la paroi du canal intramédullaire.

17. Procédé selon la revendication 16, dans lequel l'extrémité du segment d'os dérivé du canal intramédullaire est usiné pour former une surface sensiblement plane pour obtenir un segment d'os sensiblement en « forme de rondelle » composé essentiellement d'os cortical.

18. Procédé selon la revendication 17, dans lequel ledit canal est formé dans une forme asymétrique par alésage dudit canal pour former ladite forme asymétrique dans le segment d'os.

19. Procédé selon la revendication 18, dans lequel ladite forme asymétrique est sensiblement en forme de « D ».

20. Procédé selon la revendication 18, dans lequel ledit segment d'os ayant un canal sensiblement en forme de D est encore machiné de sorte que le profil externe du segment d'os s'accorde sensiblement au profil dudit canal.

21. Procédé selon la revendication 18, dans lequel ledit usinage comprend la mise en contact du segment d'os avec une meule de forme asymétrique.

22. Procédé selon la revendication 18, dans lequel ledit usinage supplémentaire comprend le montage dudit segment d'os sur un pivot fixé sur une came asymétrique et contenant le segment d'os ainsi monté, avec un cutter en rotation autour d'un axe symétrique de sorte que le cutter est constitué pour couper plus ou moins d'os comme dicté par la forme de ladite came asymétrique.

23. Procédé selon la revendication 22, comprenant en outre, l'empilement dudit segment d'os, avant ou après ledit usinage, le forage de trous dans celui=ci et le chevillage desdits segments d'os empilés les unes avec les autres.

24. Procédé selon la revendication 14, qui comprend en outre, l'usinage d'une caractéristique externe sur la surface supérieure, la surface inférieure ou les deux surfaces de l'implant.

25. Procédé selon la revendication 24, dans lequel ladite caractéristique externe est usinée en passant ledit implant dans un alésoir ou par passage répété dudit implant sur une série de dents coupantes.
